# FASCICULE DE BREVET EUROPEEN

(11) **EP 0 721 988 B1**
(45) Date de publication et mention de la délivrance du brevet: **26.01.2000**
(21) Numéro de dépôt: 95402150.7
(22) Date de dépôt: 26.09.1995
(51) Int. Cl.: C12Q 1/68, C12P 19/34

(54) **Oligonucléotide chimère et son utilisation dans l'obtention de transcrits d'un acide nucléique**
Chimärisches Oligonukleotid und dessen Verwendung zur Herstellung von Nukleinsäure-Transkripten
Chimeric oligonucleotide and its use in obtaining nucleic acid transcripts

(30) Priorité: 26.09.1994 FR 9411455
(43) Date de publication de la demande: 17.07.1996
(73) Titulaire: BIO MERIEUX, 69280 Marcy l'Etoile (FR)
(72) Inventeur: Guillou-Bonnici, Françoise, F-69100 Villeurbanne (FR); Levasseur, Pierre, F-69002 Lyon (FR); Cleuziat, Philippe, F-69007 Lyon (FR); McAllister, William, Edison, N.J. 08817 (US); Mallet, François, F-69100 Villeurbanne (FR)
(74) Mandataire: Tonnellier, Jean-Claude

(56) Documents cités:
- EP-A- 0 587 298
- WO-A-89/06700
- WO-A-92/22663
- PROCEEDINGS OF THE NATIONAL ACADEMY OF SCIENCES USA, vol. 87, no. 5, Mars 1990 pages 1874-78, GUATELLI, J. ET AL 'isothermal, in vitro amplification of nucleic acids by a multienzyme reaction modeled after retrovirus replication'
- JOURNAL OF VIROLOGICAL METHODS , vol. 35, 1991 pages 273-86, KIEVITS T EL AL. 'nasba isothermal enzymatic in virto nucleic acid amplification optimized for the diagnosis of hiv-1 infection'

### LISTE DE SEQUENCES

(1) INFORMATIONS GENERALES
   (i) DEPOSANT :
      (A) NOM : BIO MERIEUX
      (B) RUE : Chemin de l'Orme
      (C) VILLE : Marcy l'Etoile
      (E) PAYS : France
      (F) CODE POSTAL : 69280
      (G) TELEPHONE : 78872000
      (H) TELECOPIE : 78872090
   (ii) TITRE DE L' INVENTION : Oligonucléotide chimère et son utilisation dans l'obtention de transcrits d'un acide nucléique.
   (iii) NOMBRE DE SEQUENCES : 36
   (iv) FORME DECHIFFRABLE PAR ORDINATEUR :
      (A) TYPE DE SUPPORT : Disquette 3 ½
      (B) ORDINATEUR: PC compatible
      (C) SYSTEME D' EXPLOITATION : PC-DOS/MS-DOS
      (D) LOGICIEL : MSWORD 6.0 pour MS-DOS 6.20 / WINDOWS 3.10
   (v) DONNEES RELATIVES A LA DEMANDE EN CAUSE :
      (A) NUMERO DE LA DEMANDE: EP 95402150.7
(2) INFORMATIONS POUR LA SEQ ID NO: 1:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 1:
   TATTAACCCT CACTAAA 17
(2) INFORMATIONS POUR LA SEQ ID NO: 2:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 2:
   TAATACGACT CACTATA 17
(2) INFORMATIONS POUR LA SEQ ID NO: 3:
   (i) CARACTERISTIQUES DE LA SEQUENCE:
      (A) LONGUEUR: 17
      (B) TYPE: nucléotide
      (C) NOMBRE DE BRINS : simple
      (D) CONFIGURATION: linéaire
   (ii) TYPE DE MOLECULE: ADN
   (iii) HYPOTHETIQUE: NON
   (vi) ORIGINE : synthèse chimique
   (xi) DESCRIPTION DE LA SEQUENCE: SEQ ID NO: 3:
   ATTTAGGTGA CACTATA 17
(2) INFORMATIONS POUR LA SEQ ID NO: 4:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 4:
   AATTAGGGCA CACTATA 17
(2) INFORMATIONS POUR LA SEQ ID NO: 5:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 5:
   TAATACGACT CACTAAT 17
(2) INFORMATIONS POUR LA SEQ ID NO: 6:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ARN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 6:
   UGCCAUAACC AUGAGUG 17
(2) INFORMATIONS POUR LA SEQ ID NO: 7:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 7:
   TGCCATAACC ATGAGTG 17
(2) INFORMATIONS POUR LA SEQ ID NO: 8:
   (i) CARACTERISTIQUES DE LA SEQUENCE:
      (A) LONGUEUR: 50
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 8:
   TAATACGACT CACTATAGGG TTGGCCGCAG TGTTCACTCA TGGTTATGGC 50
(2) INFORMATIONS POUR LA SEQ ID NO: 9:
   (i) CARACTERISTIQUES DE LA SEQUENCE:
      (A) LONGUEUR: 19
      (B) TYPE: nucléotide
      (C) NOMBRE DE BRINS : simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (ix) CARACTERISTIQUE
      (D) AUTRES INFORMATIONS : N représente un bras 3'-C₃NH₂
   (xi) DESCRIPTION DE LA SEQUENCE: SEQ ID NO: 9:
   TAATACGACT CACTATAGN 19
(2) INFORMATIONS POUR LA SEQ ID NO: 10:
   (i) CARACTERISTIQUES DE LA SEQUENCE:
      (A) LONGUEUR: 52
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (ix) CARACTERISTIQUE
      (D) AUTRES INFORMATIONS : N représente un bras 3'-C₃NH₂
   (xi) DESCRIPTION DE LA SEQUENCE: SEQ ID NO: 10:
   ATTTAGGTGA CACTATAGAA GTTGGCCGCA GTGTTCACTC ATGGTTATGG CN 52
(2) INFORMATIONS POUR LA SEQ ID NO: 11:
   (i) CARACTERISTIQUES DE LA SEQUENCE:
      (A) LONGUEUR: 19
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (ix) CARACTERISTIQUE
      (D) AUTRES INFORMATIONS : N représente un bras 3'-C₃NH₂
   (xi) DESCRIPTION DE LA SEQUENCE: SEQ ID NO: Il:
   ATTTAGGTGA CACTATAGN 19
(2) INFORMATIONS POUR LA SEQ ID NO: 12:
   (i) CARACTERISTIQUES DE LA SEQUENCE:
      (A) LONGUEUR: 51
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (ix) CARACTERISTIQUE
      (D) AUTRES INFORMATIONS : N représente un bras 3'-C₃NH₂
   (xi) DESCRIPTION DE LA SEQUENCE: SEQ ID NO: 12:
   AATTAACCCT CACTAAAGGG TTGGCCGCAG TGTTCACTCA TGGTTATGGC N 51
(2) INFORMATIONS POUR LA SEQ ID NO: 13:
   (i) CARACTERISTIQUES DE LA SEQUENCE:
      (A) LONGUEUR: 19
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (ix) CARACTERISTIQUE
      (D) AUTRES INFORMATIONS : N représente un bras 3'-C₃NH₂
   (xi) DESCRIPTION DE LA SEQUENCE: SEQ ID NO: 13:
   AATTAACCCT CACTAAAGN 19
(2) INFORMATIONS POUR LA SEQ ID NO: 14:
   (i) CARACTERISTIQUES DE LA SEQUENCE:
      (A) LONGUEUR: 51
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (si) DESCRIPTION DE LA SEQUENCE: SEQ ID NO: 14:
(2) INFORMATIONS POUR LA SEQ ID NO: 15:
   (i) CARACTERISTIQUES DE LA SEQUENCE:
      (A) LONGUEUR: 50
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 15:
   GCCATAACCA TGAGTGAACA CTGCGGCCAA CCCTATAGTG AGTCGTATTA 50
(2) INFORMATIONS POUR LA SEQ ID NO: 16:
   (i) CARACTERISTIQUES DE LA SEQUENCE:
      (A) LONGUEUR: 50
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 16:
   GCCATAACCA TGAGTGAACA CTGCGGCCAA CCCTTTAGTG AGGGTTAATT 50
(2) INFORMATIONS POUR LA SEQ ID NO: 17:
   (i) CARACTERISTIQUES DE LA SEQUENCE:
      (A) LONGUEUR: 50
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: misc_RNA
      (D) AUTRES INFORMATIONS :
         - les nucléotides 1 à 16 sont des ribonucléotides
         - les nucléotides 17 à 50 sont des désoxyribonucléotides
      (xi) DESCRIPTION DE LA SEQUENCE: SEQ ID NO: 17:
   GCCAUAACCA UGAGUGAACA CTGCGGCCAA CCCTTTAGTG AGGGTTAATT 50
(2) INFORMATIONS POUR LA SEQ ID NO: 18:
   (i) CARACTERISTIQUES DE LA SEQUENCE:
      (A) LONGUEUR: 51
      (B) TYPE: nucléotide
      (C) NOMBRE DE BRINS : simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: misc_RNA
      (D) AUTRES INFORMATIONS :
         - les nucléotides 1 à 16 sont des ribonucléotides
         - les nucléotides 17 à 51 sont des désoxyribonucléotides
   (xi) DESCRIPTION DE LA SEQUENCE: SEQ ID NO: 18:
   GCCAUAACCA UGAGUGAACA CTGCGGCCAA CCCTATAGTG AGTCGTATTA A 51
(2) INFORMATIONS POUR LA SEQ ID NO: 19:
   (i) CARACTERISTIQUES DE LA SEQUENCE:
      (A) LONGUEUR: 51
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: misc_RNA
      (D) AUTRES INFORMATIONS :
         - les nucléotides 1 à 16 sont des ribonucléotides
         - les nucléotides 17 à 51 sont des désoxyribonucléotides
   (xi) DESCRIPTION DE LA SEQUENCE: SEQ ID NO: 19:
   GCCAUAACCA UGAGUGAACA CTGCGGCCAA CTTCTATAGT GTCACCTAAA T 51
(2) INFORMATIONS POUR LA SEQ ID NO: 20:
   (i) CARACTERISTIQUES DE LA SEQUENCE:
      (A) LONGUEUR: 50
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: mise_RNA
         (D) AUTRES INFORMATIONS :
         - les nucléotides 1 à 33 sont des ribonucléotides
         - les nucléotides 34 à 50 sont des désoxyribonucléotides
      (xi) DESCRIPTION DE LA SEQUENCE: SEQ ID NO: 20:
   GCCAUAACCA UGAGUGAACA CUGCGGCCAA CCCTTTAGTG AGGGTTAATT 50
(2) INFORMATIONS POUR LA SEQ ID NO: 21:
   (i) CARACTERISTIQUES DE LA SEQUENCE:
      (A) LONGUEUR: 34
      (B) TYPE: nucléotide
      (C) NOMBRE DE BRINS: simple
      (D) CONFIGURATION: linéaire
   (ii) TYPE DE MOLECULE: ARN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 21:
   AGAGAAUUAU GCAGUGCUGC CAUAACCAUG AGUG 34
(2) INFORMATIONS POUR LA SEQ ID NO: 22:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 22:
   AGAGAATTAT GCAGTGC 17
(2) INFORMATIONS POUR LA SEQ ID NO: 23:
   (i) CARACTERISTIQUES DE LA SEQUENCE:
      (A) LONGUEUR: 16
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 23:
   TACTGTCATG CCATCC 16
(2) INFORMATIONS POUR LA SEQ ID NO: 24:
   (i) CARACTERISTIQUES DE LA SEQUENCE:
      (A) LONGUEUR: 57
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 24:
   AAGTAAGTTG GCCGCAGTGT TATCACTCAT GGTTATGGCA GCACTGCATA 50
   ATTCTCT 57
(2) INFORMATIONS POUR LA SEQ ID NO: 25:
   (i) CARACTERISTIQUES DE LA SEQUENCE:
      (A) LONGUEUR: 51
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 25:
   TAATACGACT CACTATAGGG ATGGCATGAC AGTAAGAGAA TTATGCAGTG C 51
(2) INFORMATIONS POUR LA SEQ ID NO: 26:
   (i) CARACTERISTIQUES DE LA SEQUENCE:
      (A) LONGUEUR: 46
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: misc_RNA
      (D) AUTRES INFORMATIONS :
         - les nucléotides 1 à 29 sont des désoxyribonucléotides
         - les nucléotides 30 à 46 sont des ribonucléotides
   (xi) DESCRIPTION DE LA SEQUENCE: SEQ ID NO: 26:
   TAATACGACT CACTATAGGG CATGACAGTA AGAGAAUUAU GCAGCA 46
(2) INFORMATIONS POUR LA SEQ ID NO: 27:
   (i) CARACTERISTIQUES DE LA SEQUENCE:
      (A) LONGUEUR: 47
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: misc_RNA
      (D) AUTRES INFORMATIONS :
         - les nucléotides 1 à 30 sont des désoxyribonucléotides
         - les nucléotides 31 à 47 sont des ribonucléotides
   (xi) DESCRIPTION DE LA SEQUENCE: SEQ ID NO: 27:
   TAATACGACT CACTATAGGG CAGTGTTATC ACUCAUGGUU AUGGCUA 47
(2) INFORMATIONS POUR LA SEQ ID NO: 28:
   (i) CARACTERISTIQUES DE LA SEQUENCE:
      (A) LONGUEUR: 51
      (B) TYPE: nucléotide
      (C) NOMBRE DE BRINS : simple
      (D) CONFIGURATION: linéaire
   (ii) TYPE DE MOLECULE: ARN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 28:
   AGAGAAUUAU GCAGUGCUGC CAUAACCAUG AGUGAUAACA CUGCGGCCAA C 51
(2) INFORMATIONS POUR LA SEQ ID NO: 29:
   (i) CARACTERISTIQUES DE LA SEQUENCE:
      (A) LONGUEUR: 16
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 29:
   GCACTGCATA ATTCTT 16
(2) INFORMATIONS POUR LA SEQ ID NO: 30:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 30:
   CACTCATGGT TATGGCA 17
(2) INFORMATIONS POUR LA SEQ ID NO: 31:
   (i) CARACTERISTIQUES DE LA SEQUENCE:
      (A) LONGUEUR: 50
      (B) TYPE: nucléotide
      (C) NOMBRE DE BRINS : simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: misc_RNA
      (D) AUTRES INFORMATIONS :
         - les nucléotides 1 à 34 sont des désoxyribonucléotides
         - les nucléotides 35 à 50 sont des ribonucléotides
   (xi) DESCRIPTION DE LA SEQUENCE: SEQ ID NO: 31:
   TAATTCGACT CACTATAGGG GTCCTCCGAT CGTTGUCAGA AGUAAGUUGC 50
(2) INFORMATIONS POUR LA SEQ ID NO: 32:
   (i) CARACTERISTIQUES DE LA SEQUENCE:
      (A) LONGUEUR: 51
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "chimère ADN-ARN"
   (iii) HYPOTHETIQUE: NON
   (iv) ANTI-SENS: NON
   (vi) ORIGINE : synthèse chimique
   (ix) CARACTERISTIQUE:
      (A) NOM/CLE: misc_RNA
      (D) AUTRES INFORMATIONS :
         - les nucléotides 1 à 34 sont des désoxyribonucléotides
         - les nucléotides 35 à 51 sont des ribonucléotides
   (xi) DESCRIPTION DE LA SEQUENCE: SEQ ID NO: 32:
   TAATACGACT CACTATAGGG TTACGGATGG CATGACAGUA AGAGAAUUAC C 51
(2) INFORMATIONS POUR LA SEQ ID NO: 33:
   (i) CARACTERISTIQUES DE LA SEQUENCE:
      (A) LONGUEUR: 102
      (B) TYPE: nucléotide
      (C) NOMBRE DE BRINS: simple
      (D) CONFIGURATION: linéaire
   (ii) TYPE DE MOLECULE: ARN
   (iii) HYPOTHETIQUE: NON
   (iv) ANTI-SENS: NON
   (vi) ORIGINE : synthèse chimique
   (xi) DESCRIPTION DE LA SEOUENCE: SEQ ID NO: 33:
(2) INFORMATIONS POUR LA SEQ ID NO: 34:
   (i) CARACTERISTIQUES DE LA SEQUENCE:
      (A) LONGUEUR: 102
      (B) TYPE: nucléotide
      (C) NOMBRE DE BRINS: simple
      (D) CONFIGURATION: linéaire
   (ii) TYPE DE MOLECULE: ARN
   (iii) HYPOTHETIQUE: NON
   (iv) ANTI-SENS: NON
   (vi) ORIGINE : synthèse chimique
   (xi) DESCRIPTION DE LA SEQUENCE: SEQ ID NO: 34:
   GGGGUCCUCC GAUCGUUGUC AGAAGUAAGU UGGCCGCAGU GUUAUCACUC 50
   AUGGUUAUGG CAGCACUGCA UAAUUCUCUU ACUGUCAUGC CAUCCGUAAC 100
   CC 102
(2) INFORMATIONS POUR LA SEQ ID NO: 35:
   (i) CARACTERISTIQUES DE LA SEQUENCE:
      (A) LONGUEUR: 17
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (ix) CARACTERISTIQUE
      (D) AUTRES INFORMATIONS : N représente un bras 5'-aminolink 2
   (si) DESCRIPTION DE LA SEQUENCE: SEQ ID NO: 35:
(2) INFORMATIONS POUR LA SEQ ID NO: 36:
   (i) CARACTERISTIQUES DE LA SEQUENCE:
      (A) LONGUEUR: 18
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (iv) ANTI-SENS: NON
   (vi) ORIGINE : synthèse chimique
   (ix) CARACTERISTIQUE
      (D) AUTRES INFORMATIONS : N représente un bras 5'-aminolink 2
   (xi) DESCRIPTION DE LA SEQUENCE: SEQ ID NO: 36:
   NGTTGGCCGC AGTGTTAT 18

## Description

La présente invention a pour objet de nouveaux oligonucléotides chimères permettant la transcription et l'amplification de séquences d'acide nucléique cibles, ainsi qu'un procédé pour l'amplification d'acides nucléiques par transcription.

Il est souvent nécessaire, dans les technologies relatives aux acides nucléiques et au matériel génétique, de déterminer si un gène, une partie de gène ou une séquence nucléotidique est présent dans un organisme vivant, un extrait cellulaire de cet organisme ou tout autre échantillon biologique.

L'intérêt de la recherche de séquences nucléotidiques spécifiques est immense, notamment pour la détection d'organismes pathogènes, la détermination de la présence d'allèles, la détection de la présence de lésions dans un génome hôte et la détection de la présence d'un ARNm particulier ou de la modification d'un hôte cellulaire. Les maladies génétiques telles que la maladie de Huntington, la myopathie de Duchenne, la phénylcétonurie et la β-thalassémie peuvent être diagnostiquées par le biais de l'analyse de l'ADN des individus. De plus, le diagnostic ou l'identification des virus, viroïdes, bactéries, champignons, protozoaires ou quelque autre forme de vie végétale ou animale peut être réalisé par des expériences d'hybridation avec des sondes nucléiques.

Différents types de méthodes de détection des acides nucléiques sont décrits dans la littérature. Ces méthodes, et particulièrement celles qui requièrent la détection de polynucléotides, reposent sur les propriétés d'appariement des brins complémentaires d'acides nucléiques dans les duplex ADN-ADN, ADN-ARN et ARN-ARN par l'établissement de liaisons hydrogène entre les bases adénine et thymine (A-T) et les bases guanine et cytosine (G-C) de l'ADN double brin, ou encore entre les bases adénine et uracile (A-U) dans les duplex ADN-ARN ou ARN-ARN. L'appariement de brins d'acides nucléiques est communément appelée "hybridation d'acides nucléiques" ou simplement "hybridation".

Dans les quelques exemples cités précédemment, après avoir identifié une séquence spécifique d'un organisme ou d'une maladie, il convient d'extraire les acides nucléiques d'un échantillon, et de déterminer si cette séquence (aussi appelée "cible") est présente. De nombreuses méthodes de détection ont été développées dans ce but.

Pour la mise en oeuvre de l'invention, il est généralement nécessaire qu'une ou plusieurs séquences spécifiques de la cible aient été préalablement identifiées. La méthode la plus directe pour détecter la présence d'une séquence cible dans un échantillon d'acides nucléiques est d'obtenir une "sonde" dont la séquence est suffisamment complémentaire d'une partie de l'acide nucléique cible pour s'hybrider à celui-ci. La sonde ainsi synthétisée peut être mise en présence d'un échantillon contenant des acides nucléiques, et si la séquence cible est présente, la sonde s'hybridera et formera un produit de réaction. En l'absence de séquence cible et en évitant tout phénomène d'hybridation non spécifique, aucun produit de réaction ne sera formé. Si la sonde synthétisée est couplée à un marqueur détectable, le produit de réaction peut être détecté en mesurant la quantité de marqueur présent. Le transfert de type Southern (SOUTHERN E.M., *J*. *Mol. Biol.,* **98**, 503 (1975)) ou Northern ou la technique du Dot blot ou l'hybridation sandwich (DUNN A.R. et HASSEL J. A, *Cell,* **12**, 23, (1977)) constituent des exemples de méthodes utilisables.

La principale difficulté de cette approche est, cependant, qu'elle n'est pas directement applicable aux cas où le nombre de copies de la séquence cible présente dans un échantillon est faible (c'est à dire inférieur à 10⁷ ). Dans ces conditions, il est difficile de distinguer un signal significatif supérieur au bruit de fond de la réaction (c'est à dire de distinguer la fixation spécifique d'une sonde sur sa séquence cible de la fixation non spécifique entre la sonde et une séquence différente de la séquence cible). Une des solutions à ce problème consiste à augmenter le signal de détection par une réaction supplémentaire. En conséquence, diverses méthodes ont été décrites afin d'accroître la puissance de détection de ces techniques d'hybridation. Ces méthodes dites "d'amplification" peuvent être classés en trois catégories : l'amplification de cible, de sonde ou de signal. Les articles de Lewis (1992. *Geiretic Engineering News* **12** : 1-9) d'une part, et d'Abramson et Myers (1993. *Curr. Opin. Biotechnol*. **4** : 41-47), d'autre part, constituent de bonnes revues générales de ces méthodes.

L'amplification de cible consiste à multiplier de manière spécifique un fragment d'acide nucléique présent dans un échantillon. Elle permet d'augmenter considérablement le nombre de copies d'une séquence nucléique cible à détecter.

Les techniques d'amplification de cible décrites dans la littérature reposent principalement soit sur la répétition de cycles de synthèse d'ADN *in vitro* par élongation d'amorces nucléotidiques hybridées sur la séquence cible à amplifier par une ADN polymérase (méthode d'amplification en chaîne par polymérase, dite PCR : voir brevets des États Unis d'Amérique n°4 683 195, 4 683 202 et 4 800 159 ; demande de brevet Européen n° 0 201 184 ; méthode dite "Repair Chain Reaction", ou RCR : voir demande de brevet n° WO 90/01069; méthode dite d'amplification par déplacement de brin, ou "Strand Displacement Amplification" (SDA) : voir demande de brevet Européen n° 0 497 272; méthode d'amplification par déplacement de brin utilisant une exonucléase, ou "exonuclease-mediated strand displacement amplification" : voir demande de brevet Européen n° 0 500 224), soit sur la répétition de cycles de synthèse d'ARN *in vitro*, par réaction de transcription à l'aide d'une ARN polymérase.

Plusieurs de ces méthodes d'amplification de cible basées sur l'amplification de transcrits ont été décrites. La méthode dite TAS, décrite dans la demande de brevet n° WO 88/10315, consiste en la répétition d'un cycle de trois étapes. La première étape permet de synthétiser un ADNc à partir d'ARN en présence d'une transcriptase inverse et d'une amorce désoxynucléotidique contenant une séquence spécifique de promoteur d'ARN polymérase phagique. Suite à la dénaturation thermique de l'hétéroduplex ARN/ADNc, le brin monocaténaire d'ADNc est répliqué par la transcriptase inverse en présence d'une amorce oligonucléotidique anti-sens. L'homoduplex d'ADN ainsi obtenu lors de cette deuxième étape contient un promoteur double brin sur lequel une ARN polymérase ADN-dépendante phagique peut se fixer. La troisième étape est une transcription, conduisant à l'obtention de 30 à 1000 molécules d'ARN par matrice qui pourront à leur tour servir de matrice pour la synthèse d'ADNc et poursuivre ainsi le cycle d'amplification (DAVIS *et al*., 1990. *J*. *Infect. Dis.* **162** : 13-20).

Il existe différentes méthodes dérivées de la TAS, dont la réplication de séquence auto-entretenue, dite "Self-Sustained Sequence Replication" (ou 3SR), décrite dans la demande de brevet WO 90/06995 et la demande de brevet Européen n° 0 373 960, la méthode dite "Nucleic Acid Sequence-Based Amplification" (ou NASBA) décrite dans la demande de brevet WO 91/02818 et la demande de brevet Européen n° 0 329 822 et la méthode de réplication de séquence à l'aide d'une seule amorce dite "Single Primer Sequence Replication" (ou SPSR) décrite dans le brevet des États Unis d'Amérique n° 5 169 766. Ces méthodes possèdent en commun l'association de trois activités enzymatiques : ADN polymérase ARN- et ADN-dépendante (transcriptase inverse), ribonucléase H (RNase H ; enzyme d'Escherichia coli et/ou activité enzymatique associée de la transcriptase inverse) et ARN polymérase ADN-dépendante (ARN polymérase du bactériophage T7). Ces méthodes sont basées sur le même principe et s'effectuent à une température fixe (de 37 à 45°C), selon un processus continu de réactions de transcription inverse et de transcription afin de répliquer une cible d'ARN par l'intermédiaire d'ADNc. Comme dans le cas de la TAS, un site de fixation d'une ARN polymérase (phage T7) est introduit dans l'ADNc par l'amorce utilisée pour l'étape de transcription inverse. Néanmoins, la dénaturation de l'hétéroduplex ARN/ADNc est effectuée de façon isotherme par hydrolyse spécifique de l'ARN de cet hétéroduplex par l'activité RNase H. L'ADNc libre est alors répliqué à partir d'une seconde amorce oligonucléotidique par la transcriptase inverse. L'homoduplex ADN/ADN est transcrit en ARN par l'ARN polymérase T7 et cet ARN peut à nouveau servir de matrice pour le cycle suivant.

Une autre méthode, dite "Ligation Activated Transcription" (ou LAT) décrite dans le brevet des États Unis d'Amérique n° 5 194 370, utilise les mêmes activités enzymatiques que les méthodes 3SR, SPSR et NASBA et fonctionne sur le même cycle. Elle diffère cependant par le mode d'installation d'une séquence promotrice qui dans ce cas est introduite sur l'extrémité de l'ADNc par ligation d'une structure tige-boucle contenant le promoteur, en présence d'une ADN ligase.

Il existe d'autres méthodes d'amplification de cible basées sur l'amplification de transcrits décrites dans la demande de brevet européen n°0 369 775. L'une diffère de la LAT uniquement par le fait que le promoteur est composé de deux oligonucléotides distincts. L'autre utilise deux activités enzymatiques, une ligase et une ARN polymérase ARN dépendante reconnaissant un promoteur ADN double brin. Le promoteur, appelé promoteur mobile, est constitué de deux oligonucléotides. L'un porte une séquence promoteur sens et une séquence sonde permettant l'hybridation sur l'extrémité 3' d'une cible ARN, l'autre porte seulement une séquence promoteur anti-sens. Par hybridation, l'extrémité 5' de l'oligonucléotide promoteur anti-sens est juxtaposée à l'extrémité 3' de la cible, puis ligaturée avec cette même extrémité. La transcription par l'ARN polymérase appropriée résulte dans la synthèse de multiples transcrits. Ces transcrits sont hybridés et ligaturés à un second promoteur mobile. La transcription de la matrice ARN résulte dans la synthèse de transcrits complémentaires. Le processus peut être réitéré, aboutissant à une amplification exponentielle de la séquence cible initiale.

Les méthodes d'amplification de cible peuvent être remplacées par des méthodes d'amplification de sonde. L'article de Birkenmeyer et Mushahwar (1991. ,*J. Vitrol*. *Meth.* **35**:117-126) fournit une revue de ces méthodes. Au sens propre du terme, elles désignent tout procédé conduisant *in vitro* à l'obtention d'un très grand nombre de copies de la sonde. L'une d'entre elles, la méthode dite "Qβ réplicase", décrite dans la demande de brevet internationale WO 87/06270, utilise une activité ARN polymérase ARN dépendante, en particulier celle de la réplicase du phage Qβ. Cette enzyme possède une grande spécificité et affinité pour l'ARN simple brin de ce phage et peut répliquer cet ARN *in vitro* (BIEBRICHER *et al*., 1986. *Nature* **321**: 98-91). La demi-vie très importante de cette enzyme permet la synthèse de nombreux brins d'ARN complémentaires correspondant aux formes positives et négatives de cette molécule d'ARN. L'exploitation de ce système de réplication repose sur la possibilité d'insérer une sonde au sein de l'ARN MDV-1, substrat naturel de la Qβ réplicase, sans altérer l'efficacité ou la spécificité de sa réplication par l'ARN polymérase du phage Qβ (LIZARDI *et al*. 1988. *BioTechnology* **6**: 1197-1202). La méthode d'amplification consiste en une hybridation de la sonde ARN recombinante sur l'acide nucléique cible, suivie d'un lavage sélectif intensif pour éliminer toute hybridation aspécifique. L'hybride formé entre la sonde ARN et la cible est alors dénaturé et la sonde ARN libérée est soumise à l'amplification par la Qβ réplicase ajoutée au milieu.

Pour toutes les méthodes décrites précédemment, diverses techniques de détection peuvent être utilisées. L'une d'entre elles consiste à détecter les produits de réaction ayant une taille définie par séparation électrophorétique. Les méthodes varient selon le procédé de séparation, qui peut impliquer la séparation sur gel, la fixation sur différentes phases solides (billes, plaque de microtitration, latex, particules magnétiques). Une autre méthode utilise le marquage d'une sonde de détection avec un radioisotope tel que le ³² P, par exemple, puis la détection de la radioactivité émise par les produits de réaction en combinaison ou non avec une électrophorèse. Une autre méthode consiste à modifier chimiquement une amorce oligonucléotidique en y ajoutant un ligand (la biotine ou la digoxigénine, par exemple), une enzyme (phosphatase alcaline, peroxydase, β-galactosidase, par exemple), un marqueur fluorescent (la phycobiliprotéine, la fluorescéine ou la rhodamine, par exemple), un marqueur luminescent (un ester d'acridinium, par exemple), des groupements à densité électronique détectables par microscopie électronique ou par des méthodes électriques (conductivité, ampérométrie, voltamétrie, mesures d'impédance par exemple), des groupements détectables par des méthodes optiques (diffraction, résonance plasmon de surface, variation d'angle de contact par exemple) ou par des méthodes physiques (spectroscopie de force atomique, effet tunnel par exemple) ou une combinaison de ces modifications. Une autre méthode consiste à développer une amorce nucléotidique de détection qui s'hybridera sur le produit de réaction d'amplification et sera étendu par une polymérase en présence de ribonucléosides triphosphates (cette amorce peut dans ce cas être également modifiée comme décrit précédemment). Toutes ces méthodes peuvent être adaptées pour être mises en oeuvre sur phase solide comme en solution (phase homogène).

Néanmoins, toutes les techniques d'amplification précédemment présentées possèdent au moins une limitation importante. Elles ne permettent d'obtenir un produit d'amplification qu'à partir d'un seul type d'acide nucléique cible : ARN ou ADN. Dans certains cas tels que la PCR, la LCR ou la RCR, le facteur le plus limitant est la nécessité de réaliser de nombreux cycles de températures afin de dissocier les produits de réaction de la cible. Ceci limite le choix des enzymes utilisables aux enzymes thermostables. De plus, la réalisation de tels cycles successifs de température constitue une contrainte pour l'automatisation de ces techniques. Un autre inconvénient de certaines techniques d'amplification est la limitation de la taille du produit de réaction d'amplification. Les techniques telles que la RCR ou la LCR ne permettent d'amplifier que la séquence de la cible correspondant aux amorces et aux sondes nucléotidiques utilisées dans le processus d'amplification. Le bruit de fond non spécifique (c'est à dire en absence de la cible) est également un sérieux inconvénient de certaines techniques : dans le cas de la LCR par exemple, une ligation des extrémités des oligonucléotides libres en excès s'effectue même en l'absence de la cible. D'autres méthodes telles que la SDA sont limitées dans le type de séquence cible à amplifier, puisque cette séquence ne doit pas comporter de site de restriction correspondant à l'endonucléase utilisée dans le procédé, et il est donc indispensable de connaître sinon la séquence nucléique totale du fragment à amplifier au moins la carte de restriction dudit fragment. Cette limitation est encore accrue par le fait que le choix des endonucléases de restriction est restreint à celles ayant la capacité de cliver un site de reconnaissance comportant des nucléotides modifiés. Outre les limitations concernant le choix du nucléotide modifié, dues à la synthèse chimique, le procédé d'amplification est également limité par son rendement puisqu'il est connu que le Km des polymérases pour les nucléotides modifiés est supérieur à celui pour les nucléotides naturels pour les polymérases, d'où une plus faible efficacité d'incorporation enzymatique des nucléotides modifiés dans la cible à amplifier. Un autre inconvénient de certaines techniques d'amplification réside dans le nombre élevé d'activités enzymatiques impliquées dans le processus d'amplification. Les méthodes dérivées de la TAS, telles que la 3SR ou la NASBA requièrent au moins quatre activités enzymatiques (ADN polymérase ADN dépendante, ADN polymérase ARN dépendante, ARN polymérase ADN dépendante, RNase H), voire cinq dans le cas de la LAT (ADN ligase en plus). Il est par conséquent très difficile de parvenir à des conditions réactionnelles satisfaisant simultanément ces quatre ou cinq activités enzymatiques. De plus, les techniques transcriptionnelles ne permettent une amplification qu'à partir de molécules cibles ARN mais pas ADN. Enfin, si diverses techniques d'amplification ont recours à des activités de nucléases (exonucléase, endonucléase, RNase) comme moyen de séparation de brins d'acides nucléiques (3SR, NASBA, demande de brevet Européen n° 0 500 224), leur utilisation est néanmoins délicate, car il est indispensable d'obtenir une action ménagée et rigoureusement contrôlée de ces enzymes afin de maintenir un équilibre entre les différentes activités enzymatiques impliquées.

Par ailleurs, les techniques actuellement connues d'amplification exponentielle utilisant des ARN polymérases ARN dépendantes ont un potentiel bien supérieur aux autres techniques, puisqu'elles n'utilisent qu'une ou deux enzymes et ne nécessitent pas de dénaturation. Cependant, ces méthodes présentent encore des lacunes. La méthode de la Qβ réplicase ne permet pas l'amplification directe de la cible, car la spécificité de l'enzyme pour sa matrice est liée à une structure ARN très complexe, d'installation difficile sur une cible. Quant à la méthode du promoteur mobile appliquée sur matrice ARN (décrite dans la demande de brevet EP 0 369 775), elle nécessite à chaque cycle l'installation de deux oligonucléotides par ligation sur la cible pour former un promoteur opérationnel.

Compte tenu de l'analyse précédente, la conception de nouvelles méthodes d'amplification apparaît souhaitable, et la présente invention a notamment pour objet une méthode d'amplification cyclique d'une séquence nucléotidique cible avec formation de transcrits, de mise en oeuvre simple, ne nécessitant que deux oligonucléotides, et pouvant fonctionner de manière isotherme.

Avant de définir plus complètement l'invention, on donne ci-après la définition de quelques termes qui seront employés dans la suite de la description.

Les termes "fragment d'acide nucléique", "segment d'acide nucléique" ou "oligonucléotide" sont utilisés indifféremment dans la présente demande pour désigner une séquence polynucléotidique d'ADN, d'ARN ou de chimère ADN/ARN, contenant éventuellement un ou plusieurs nucléotides modifiés. Une telle séquence polynucléotidique possède généralement une "longueur" d'au moins 5 désoxyribonucléotides et/ou ribonucléotides, pouvant contenir éventuellement au moins un nucléotide modifié. Une séquence polynucléotidique modifiée comprend par exemple au moins une base modifiée telle que l'inosine, la méthyl-5-désoxycytidine, la diméthylamino-5-désoxyuridine, la désoxyuridine, la diamino-2,6-purine, la bromo-5-désoxyuridine, la pseudouridine, la pseudoisocytidine ou toute autre base modifiée permettant l'hybridation. La séquence polynucléotidique peut aussi être modifiée au niveau des liaisons internucléotidiques (pouvant impliquer par exemple des liaisons phosphorothioate, H-phosphonate, alkyl phosphonate), ou au niveau du squelette comme c'est le cas par exemple pour les alpha-oligonucléotides (demande de brevet français n° 2 607 507) ou les PNA (EGHOLM *et al*., 1992. *J*. *Am. Chem. Soc.* **114** : 1895-1897). Dans une séquence polynucléotidique modifiée, plusieurs modifications telles qu'indiquées ci-dessus peuvent être présentes en combinaison.

Le terme "support solide" tel qu'utilisé ici inclut tous les matériaux sur lesquels peut être immobilisé un fragment d'acide nucléique pour une utilisation dans les méthodes utilisant l'hybridation (notamment les tests diagnostiques), en chromatographie d'affinité et dans les processus de séparation. Des matériaux naturels ou de synthèse, poreux ou non, magnétiques ou non, modifiés chimiquement ou non, peuvent constituer le support solide, notamment des polysaccharides tels que les matériaux à base de cellulose, par exemple du papier ; des dérivés de cellulose tels que l'acétate de cellulose et la nitrocellulose ; des polymères tels que le poly(chlorure de vinyle), le polyéthylène, le polystyrène, les polyacrylates ou des copolymères tels que des copolymères de chlorure de vinyle et de propylène, de chlorure de vinyle et d'acétate de vinyle, etc ; des polymères de type poly(N-isopropylacrylamide) ou en abrégé NIPPAM ; des copolymères à base de styrène ou de styrènes substitués; des fibres naturelles telles que le coton et des fibres synthétiques telles que le Nylon ; des céramiques ; de la silice. Les supports solides selon l'invention peuvent être, par exemple, sous la forme d'une plaque de microtitration, d'une feuille, d'un cône, d'un tube, d'un puits, d'une bille ou analogue.

Le terme "hybridation" désigne la formation de duplex entre des séquences nucléotidiques qui sont suffisamment complémentaires, par appariement de bases de type "Watson et Crick" ou de type "Hoogsteen" (Thuong, N.T., Angew. Chem. Int. Ed. Engl., 32, 666-690, 1993).

Le terme "oligonucléotide" désigne une séquence simple brin composée de nucléotides qui peuvent être des désoxyribonucléotides ou des ribonucléotides, ces nucléotides pouvant être modifiés comme décrit précédemment dans le paragraphe relatif à la description des termes "fragment d'acide nucléique" ou analogues.

Le terme "oligonucléotide chimère" désigne un oligonucléotide composé d'au moins deux variétés différentes de séquences nucléiques, les unes différant des autres par leur nature chimique. On citera par exemple des oligonucléotides chimères contenant au moins une séquence oligodésoxyribonucléotidique, ou de type ADN, et au moins une séquence oligoribonucléotidique, ou de type ARN.

Le terme "séquence promoteur" ou "région promoteur" désigne une région en 5' d'un oligonucléotide, en particulier d'un oligonucléotide chimère tel que décrit dans la présente demande, qui comporte un des brins d'un promoteur d'une ARN polymérase, c'est à dire une séquence ou une structure qui permet d'initier la transcription. De telles séquences sont bien connues. A titre d'exemple, on peut citer les promoteurs naturels des ARN polymérases, des séquences raccourcies dérivées des promoteurs naturels et ayant conservé leur fonctionnalité, ou encore les structures "boucles" capables d'initier la transcription (MOLLEGAARD N.E. *et al*,1994,*Proc*. *Natl*. *Acad. Sci.* USA,**91**, 3892-3895).

Par séquence sens d'un promoteur d'ARN polymérase, on désigne la séquence d'un promoteur dont l'extrémité 3' est localisée en amont du site d'initiation de la transcription qui est défini par ce même promoteur.

Par séquence anti-sens d'un promoteur d'ARN polymérase, on désigne la séquence d'un promoteur dont l'extrémité 5' est localisée en amont du site d'initiation de la transcription qui est défini par ce même promoteur.

Par séquence homologue d'une autre séquence, on désigne une séquence capable de s'hybrider avec une séquence strictement complémentaire de ladite autre séquence. La séquence homologue est soit identique à ladite autre séquence, soit suffisamment semblable pour s'hybrider avec ladite séquence strictement complémentaire.

Par séquence complémentaire d'une autre séquence, on désigne une séquence pouvant s'hybrider avec ladite autre séquence. Une séquence strictement complémentaire d'une autre est une séquence dans laquelle chacune des bases peut apparier avec une base de l'autre séquence, sans mésappariement.

L'expression "acide nucléique à amplifier" peut désigner indifféremment l'un des deux brins complémentaires d'un acide nucléique cible dont on désire multiplier le nombre de copies.

Le terme "séquence d'initiation" ou "région d'initiation" désigne une région des oligonucléotides chimères décrits dans la présente demande, qui est comprise entre la région promoteur située du côté de l'extrémité 5' et la région sonde située du côté de l'extrémité 3'. La nature chimique de la séquence d'initiation permet une interaction spécifique soit avec l'acide nucléique à amplifier ou réamplifier, soit avec sa séquence complémentaire. Le rôle de cette séquence d'initiation est de permettre, par sa nature chimique et sa séquence au moins partiellement complémentaire de l'acide nucléique à amplifier ou réamplifier, la dégradation sélective de la séquence cible et/ou de la séquence complémentaire de la séquence cible dans le duplex séquence cible/séquence d'initiation et/ou le duplex séquence complémentaire de la séquence cible/séquence d'initiation, respectivement, notamment par digestion par la RNase H si la séquence cible est un ARN. Par ailleurs, la séquence d'initiation renferme le site d'initiation de la transcription. Les transcrits générés sous l'influence de la région promoteur porteront en 5' une séquence qui sera complémentaire d'au moins une partie de la séquence d'initiation et dont la longueur sera fonction de la localisation du site d'initiation de la transcription sur la séquence d'initiation.

Le terme "séquence sonde" ou "région sonde" désigne une région des oligonucléotides chimères décrits dans la présente demande, cette région étant située du côté de l'extrémité 3' par rapport à la région d'initiation. La nature chimique de la séquence sonde permet l'hybridation de l'oligonucléotide chimère de façon spécifique et stable sur l'acide nucléique à amplifier ou réamplifier, notamment lorsque celui-ci est de nature ribonucléique, par la formation d'un duplex insensible à l'activité de dégradation mentionnée dans la définition précédente. Par ailleurs, l'extrémité 3' de cette séquence sonde sera obligatoirement bloquée pour éviter son élongation par une polymérase.

Le terme "duplex" désigne un produit d'hybridation double brin acide nucléique-acide nucléique, étant entendu que l'un et/ou l'autre de ces acides nucléiques peut renfermer au moins une modification chimique.

Le terme "hétéroduplex" désigne un hybride ARN-ADN. Le terme "homoduplex" désigne un hybride ADN-ADN ou ARN-ARN.

Le terme "nucléosides triphosphates" désigne indifféremment des désoxyribonucléosides triphosphates et/ou des ribonucléosides triphosphates, éventuellement modifiés.

Dans la présente demande, l'expression "amont" désigne une région située du côté de l'extrémité 5' de l'acide nucléique ou de la séquence polynucléotidique dont il s'agit, et l'expression "aval" désigne une région située du côté de l'extrémité 3' dudit acide nucléique ou de ladite séquence polynucléotidique.

La position +1 désigne le site d'initiation de la transcription. Le nucléotide matrice en +1 est le premier nucléotide recopié, et se retrouvera en 5' de l'ARN néosynthétisé.

Le substantif "transcrit" désigne un produit de transcription, c'est-à-dire un ARN néosynthétisé lors de la transcription de la matrice sous la dépendance du promoteur ayant initié la transcription.

Un oligonucléotide "bloqué en 3'" est un oligonucléotide présentant dans la région 3' terminale une modification empêchant son élongation par une polymérase. L'oligonucléotide bloqué en 3' peut par exemple porter un nucléotide 3' terminal dépourvu du groupement 3'-OH, notamment par incorporation d'un nucléoside comme la désoxy-3'-adénosine (ou cordycépine). On peut aussi bloquer l'extrémité 3' en substituant l'hydrogène du groupement 3'-OH par exemple à l'aide d'un groupement alkyle ou aryle, de façon connue en soi. Le blocage en 3' peut encore être obtenu par la présence à l'extrémité 3' d'un ou plusieurs nucléotides qui ne peuvent pas s'apparier avec la matrice-cible, en particulier par le choix pour l'extrémité 3' d'un ou plusieurs nucléotides non complémentaires de la cible.

Une séquence oligonucléotidique est dite "de type ADN" si elle est constituée d'ADN ou s'il s'agit d'une séquence polynucléotidique modifiée possédant, outre les propriétés d'hybridation de brins des acides nucléiques, au moins une autre propriété en commun avec l'ADN. Cette propriété commune dépendra bien entendu de la fonctionnalité de la séquence modifiée : c'est dans l'exercice de cette fonctionnalité que la séquence en question a une propriété en commun avec l'ADN (c'est-à-dire : se comporte comme un ADN). Par exemple, la séquence promoteur définie précédemment peut être une séquence modifiée qui, sous forme de double brin, est capable de fonctionner comme promoteur d'ARN polymérase tout comme les promoteurs classiques constitués d'ADN double brin : on dira donc qu'une telle séquence est de type ADN. De même, la séquence d'initiation définie précédemment peut être une séquence modifiée qui d'une part peut s'hybrider avec une partie de la cible, et qui d'autre part, lorsqu'elle est hybridée avec une partie de la cible, permet la dégradation de ladite partie de la cible (tout comme un ADN hybridé à un ARN permet la dégradation de la partie hybridée de l'ARN sous l'action d'une RNase H).

Une séquence oligonucléotidique est dite "de type ARN" si elle est constituée d'ARN ou s'il s'agit d'une séquence polynucléotidique modifiée possédant, outre les propriétés d'hybridation de brins des acides nucléiques, au moins une autre propriété en commun avec l'ARN. Cette propriété commune dépend bien entendu de la fonctionnalité de la séquence modifiée dont il s'agit. Par exemple, la séquence sonde définie précédemment peut être une sonde modifiée qui est capable d'hybridation avec la cible et qui, lorsqu'elle est hybridée avec une partie de la cible constituée d'ARN, permet (comme une séquence d'ARN, et contrairement à une séquence d'ADN), d'éviter la dégradation de ladite partie de la cible sous l'action d'une RNase.

Par "extrémité définie" d'un acide nucléique on entend une extrémité de séquence connue. Cette extrémité peut être naturelle ou obtenue par modification chimique, physique ou enzymatique.

La présente invention a donc pour objet un oligonucléotide chimère pouvant notamment être utilisé dans un procédé d'amplification d'une séquence-cible d'un acide nucléique, ladite séquence-cible comprenant, du côté de son extrémité 3', une séquence aval, ledit oligonucléotide chimère comprenant successivement, de son extrémité 5' vers son extrémité 3':
- un premier segment oligonucléotidique, de type ADN, comprenant une séquence sens d'un promoteur d'une ARN polymérase,
- un second segment oligonucléotidique, de type ADN, comprenant le site d'initiation de la transcription pour ledit promoteur, au moins la région 3' dudit second segment étant capable de s'hybrider avec au moins une partie de ladite séquence aval,
- et un troisième segment oligonucléotidique, de type ARN, dont au moins la région comprenant l'extrémité 5' dudit troisième segment est capable de s'hybrider avec une partie de la séquence-cible contiguë à la partie de ladite séquence aval qui est capable d'hybridation avec ledit second segment, ledit troisième segment étant bloqué en 3'.

Généralement, les second segment et troisième segment de l'oligonucléotide peuvent contenir chacun de 2 à 30 nucléotides, et en particulier de 4 à 20 nucléotides.

Comme on le verra ci-après dans la description détaillée de l'invention, un oligonucléotide chimère tel que défini précédemment permet d'obtenir facilement des transcrits à partir d'une séquence-cible d'ADN ou d'ARN.

En utilisant deux oligonucléotides chimères tels que définis ci-après, l'un capable de s'hybrider avec une séquence-cible et l'autre capable de s'hybrider avec une séquence complémentaire de ladite séquence-cible, il est possible de réaliser une amplification cyclique de la séquence-cible et de sa séquence complémentaire, et l'invention a également pour objet un ensemble d'oligonucléotides pour l'obtention de transcrits, ou pour l'amplification cyclique avec obtention de transcrits, d'une séquence-cible d'un acide nucléique et/ou de sa séquence complémentaire, ledit ensemble comprenant :
- un premier oligonucléotide chimère, tel que défini précédemment, capable de s'hybrider avec une région aval de la séquence-cible,
- et un second oligonucléotide chimère, tel que défini précédemment, capable de s'hybrider avec une région aval d'une séquence nucléotidique complémentaire de ladite séquence-cible.

Les oligonucléotides chimères de l'invention peuvent être synthétisés selon les méthodes connues ; voir par exemple SINHA et al., Biochimie, 75, 13-23 (1993).

L'invention a également pour objet un procédé d'obtention de transcrits, ou d'amplification cyclique avec obtention de transcrits, d'une séquence-cible d'un acide nucléique, et/ou d'une séquence complémentaire de ladite séquence-cible, ladite séquence-cible comprenant à son extrémité 5' une séquence amont et à son extrémité 3' une séquence aval, ledit procédé comprenant dans des conditions permettant l'hybridation et le fonctionnement des activités enzymatiques présentes, la mise en contact d'un échantillon contenant ou susceptible de contenir ledit acide nucléique avec :
a) un premier oligonucléotide-chimère tel que défini ci-dessus, capable de s'hybrider avec la séquence aval de la séquence-cible,
b) éventuellement un second oligonucléotide-chimère, tel que défini ci-dessus, capable de s'hybrider avec une région aval d'une séquence complémentaire de ladite séquence-cible, ladite région aval étant complémentaire de ladite séquence amont,
c) et un système enzymatique contenant une activité ADN-polymérase, une activité ARN-polymérase capable de fonctionner avec ledit promoteur et une activité capable de dégrader spécifiquement la région de la cible ou du complémentaire de la cible qui est capable d'appariement avec au moins une partie du deuxième segment du premier ou du deuxième oligonucléotide, respectivement, et l'incubation pendant un temps suffisant du mélange obtenu.

Les séquences amont et aval sont des séquences non chevauchantes. Le plus souvent, les séquences amont et aval ne seront pas identiques.

Le procédé de l'invention est mis en oeuvre dans des conditions permettant l'hybridation des oligonucléotides avec leurs brins cibles et le fonctionnement des diverses activités enzymatiques impliquées. Ces conditions sont bien connues et ne seront pas rappelées en détail ici. On opère bien entendu dans des solutions tampons appropriées, en présence d'un excès de ribonucléosides triphosphates et desoxyribonucléosides triphosphates nécessaires notamment pour l'élongation de l'ADN et la synthèse des produits de transcription.

La réaction d'amplification peut aussi être effectuée sur support solide. La fixation de l'oligonucléotide chimère qui porte une séquence sonde s'hybridant avec la molécule d'acide nucléique de départ permet une étape de purification de la cible préalablement à l'étape d'amplification, et permet la détection sur support solide des seuls produits d'amplification qui ont une séquence homologue à la cible. La fixation des deux oligonucléotides chimères permet la détection sur support solide de tous les produits d'amplification, ceux ayant une séquence homologue à la cible, et ceux ayant une séquence complémentaire de la cible.

Le procédé de l'invention peut être mis en oeuvre de façon isotherme. Les spécialistes comprendront aisément qu'il est toutefois possible de mettre en oeuvre ce procédé avec utilisation de cycles de températures destinés éventuellement à favoriser une activité enzymatique aux dépens d'une autre, afin de favoriser, le cas échéant, un ordre précis dans le fonctionnement successif des diverses activités enzymatiques impliquées.

Selon un mode de réalisation particulier, ladite activité de dégradation spécifique de la cible est une activité de RNase H. Il faut bien comprendre que dans le cas où l'acide nucléique de départ est un ADN, l'activité de dégradation de la séquence-cible par la RNase H s'effectue sur les transcrits (ARN) obtenus grâce au procédé de l'invention et non sur l'ADN de départ.

Le procédé de l'invention peut être mis en oeuvre en introduisant dès le départ, dans l'enceinte réactionnelle, tous les ingrédients nécessaires à la bonne marche du procédé, ce qui permet d'éviter les contaminations.

Bien entendu, le procédé de l'invention peut impliquer des étapes préalables telles que l'extraction des acides nucléiques d'un échantillon biologique ou la dénaturation de la molécule d'acide nucléique de départ pour obtenir une structure simple brin si l'acide nucléique de départ est un duplex ou s'il s'agit d'une molécule très structurée.

De même, le procédé de l'invention peut être suivi par exemple d'étapes de séparation et/ou de quantification des produits de transcription ou des produits d'amplification, ces étapes pouvant être mises en oeuvre de façon connue en soi.

L'invention a encore pour objet un nécessaire pour la détection d'un acide nucléique cible susceptible d'être présent dans un échantillon, ledit nécessaire permettant la mise en oeuvre de la méthode d'amplification décrite précédemment.

On va maintenant décrire l'invention de façon plus détaillée en faisant référence au dessin annexé dans lequel la figure unique décrit schématiquement un mode particulier de réalisation du procédé de l'invention, comprenant l'obtention de séquences d'acides nucléiques à extrémités définies pouvant entrer dans le cycle de la réaction d'amplification encore appelée "Amplification utilisant une Chimère Promoteur" (en abrégé ACP) objet de l'invention. Les lignes ondulées représentent les acides nucléiques de nature ribonucléique. Les lignes fines représentent les acides nucléiques de nature désoxyribonucléique. Les lignes épaisses représentent les séquences d'acide désoxyribonucléique constituant l'un des brins d'une séquence capable de jouer le rôle d'un promoteur. Les deux oligonucléotides chimères sont constitués chacun d'une séquence d'acide désoxyribonucléique (P₁ ou P₂) pouvant jouer le rôle de brin sens de promoteur d'une ARN polymérase, d'une séquence désoxyribonucléique d'initiation de la transcription (I₁ ou I₂) et d'une séquence ribonucléique sonde (S₁ ou S₂). Les oligonucléotides chimères sont bloqués en 3'. Ce blocage est représenté par un point noir. Le site d'initiation de la transcription, appelé aussi position +1, est représenté par une flèche. Deux brins d'acide nucléique représentés en parallèle, côte à côte, sont hybridés. Les signes "+" et "-" font référence à des séquences appartenant à des brins opposés d'un acide nucléique, c'est à dire des brins complémentaires.

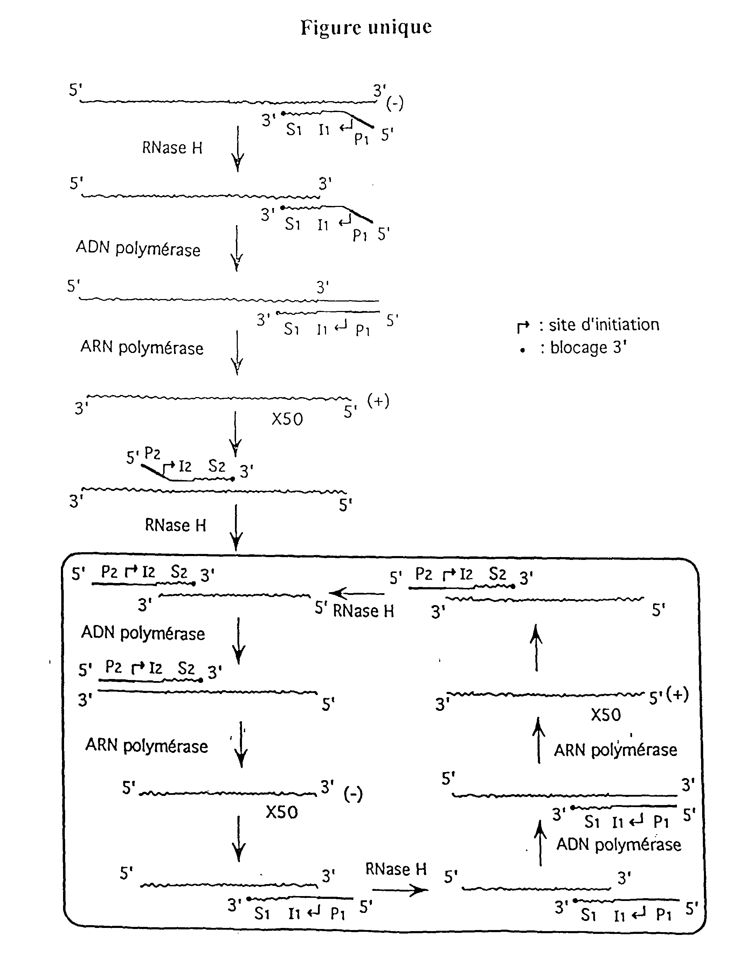

Comme on peut le vérifier aisément sur la figure 1, selon que l'on utilise un ou deux oligonucléotides chimères, le procédé de l'invention revient soit à un procédé d'obtention de transcrits, soit à une amplification cyclique avec production de transcrits.

Selon le schéma de la figure 1, le premier oligonucléotide chimère (portant les séquences P₁, I₁, S₁) est hybridé sur une molécule de départ ribonucléique, par l'intermédiaire de la séquence sonde de nature ribonucléique (S₁), et de tout ou partie de la séquence d'initiation de nature désoxyribonucléique (I₁). La dégradation de l'ARN sur la région hétéroduplex ARN:ADN formée par hybridation de la molécule de départ ARN avec tout ou partie de la séquence d'initiation ADN (I₁) permet de créer une extrémité 3' sur la molécule de départ et de définir ainsi l'extrémité 3' de la séquence cible, si elle ne l'était pas déjà. Préférentiellement, l'ARN de la région hétéroduplex ARN:ADN est dégradé par une activité RNase H. Cependant, cette dégradation peut être effectuée par tout autre moyen. La région de I₁ qui est hybridée avec la séquence cible est autant que possible une séquence adaptée à la digestion par la RNase H utilisée. En particulier, cette région devra être suffisamment longue pour servir de substrat à cette enzyme, et porter de préférence les bases les plus favorables à la réaction. Selon Keller et al (1979, *Nucleic Acid Research* **7**: 179-192), une séquence de 4 nucléotides serait suffisante pour servir de substrat à la RNase H. Dans le cas où la molécule de départ est ADN et où la RNase H est présente initialement, la séquence d'initiation devra, notamment par sa taille, permettre une hybridation stable de l'oligonucléotide chimère sur l'acide nucléique de départ. L'activité RNase H peut être portée par différentes enzymes, comme par exemple la RNase H de *E*. *coli*, ou associée à une transcriptase inverse, comme la transcriptase inverse de l'AMV (Avian Myeloblastosis Virus).

Le premier oligonucléotide chimère reste hybridé avec la molécule de départ ARN car la RNase H ne dégrade pas les homoduplex ARN:ARN (ni les ARN monobrins). La séquence sonde S1 est suffisamment longue pour permettre une hybridation stable par elle-même. La longueur de cette séquence sonde est aussi garante de la bonne spécificité du système : seul un acide nucléique portant une séquence s'hybridant spécifiquement avec la chimère sera amplifié. De préférence, la séquence sonde aura au moins 5 nucléotides. Néanmoins, on peut aussi améliorer la stabilité de l'hybridation en utilisant des nucléotides modifiés.

Une ADN polymérase ADN dépendante capable d'utiliser une amorce ARN permet l'élongation de l'ARN à partir de l'extémité 3' libérée et la synthèse du brin anti-sens du promoteur. Les ADN polymérases qui conviennent sont en particulier celles qui ont un rôle dans le cycle de réplication (comme par exemple l'ADN polymérase alpha de *P*. *polycephalum* ou l'ADN polymérase alpha de placenta humain). Cependant, les polymérases de réparation de l'ADN (comme le fragment de Klenow de l'ADN polymérase I d'*E*. *coli ,* ou l'ADN polymérase b-like de *P. polycephalum)* acceptent aussi les amorces ARN (Nevinsky *et al*, 1990, *Biochemisty* 29 : 1200-1207). On peut utiliser le fragment de Klenow de l'ADN polymérase I de *E. coli* exo (-) (dépourvu d'activité 3' - 5' exonucléase), l'ADN polymérase du bactériophage T7 (Sequenase, marque déposée), ou encore les transcriptases inverses (AMV ou MMLV). L'extrémité 3' de l'oligonucléotide chimère sonde-promoteur étant bloquée, il n'y a pas d'élongation de l'ADN polymérase. Différents modes de blocage peuvent être envisagés, comme indiqué dans les "définitions" ci-dessus.

La synthèse du brin anti-sens du promoteur permet la reconnaissance du promoteur par une ARN polymérase spécifique de ce promoteur, par exemple une ARN polymérase phagique. Lorsque le promoteur est sous forme de double brin, la transcription avec l'ARN polymérase appropriée devient possible. Le promoteur double brin peut porter par exemple la séquence d'un promoteur pour la T3, la T7, la SP6, la BA14 ou la K11 ARN polymérase, ou une séquence dérivée ayant conservé une fonctionnalité de promoteur pour ces ARN polymérases. On utilise par exemple une séquence spécifique pour la T3 ARN polymérase comme la séquence consensus SEQ ID n°1, une séquence du promoteur spécifique pour la T7 ARN polymérase comme la séquence consensus SEQ ID n°2, une séquence spécifique pour la SP6 ARN polymérase comme la séquence consensus SEQ ID n°3, une séquence spécifique pour la K11 ARN polymérase comme la séquence consensus SEQ ID n°4, ou une séquence spécifique pour la BA14 ARN polymérase comme la séquence consensus SEQ ID n°5. Par ailleurs, lorqu'on utilise deux oligonucléotides chimères, les séquences promotrices des premier et second oligonucléotides chimères peuvent être spécifiques de deux ARN polymérases distinctes.

L'activité ARN polymérase ADN dépendante est largement décrite dans la littérature pour les ARN polymérases phagiques précédemment citées. Cependant, l'invention peut nécessiter une transcription sur une matrice de nature ribonucléique et donc une activité ARN polymérase ARN dépendante initiée de façon spécifique par le promoteur. Une telle activité a été décrite pour la T3 ARN polymérase (LEARY *et al, 1991. Gene* **106**: 93-96, demande de brevet européen n° 369 775). Dans l'invention décrite par le brevet européen n° 369 775, la réaction d'amplification repose sur une activité ARN polymérase ARN dépendante avec une initiation de la transcription sur un ribonucléotide sur le brin matrice en position +1, la transcription s'effectuant sur une matrice entièrement ARN. Cependant, le rendement de cette réaction est faible. La transcription aboutit à la synthèse de moins d'un transcrit pour dix matrices. On a maintenant découvert que ce taux d'amplification peut être avantageusement augmenté lorsque l'initiation de la transcription par l'ARN polymérase ARN dépendante (par exemple la T3 ARN polymérase, la T7 ARN polymérase ou la SP6 ARN polymérase) débute avec un désoxyribonucléotide sur le brin anti-sens (position +1) et que la transcription s'effectue d'abord sur une matrice ADN complémentaire de la séquence d'initiation I puis sur une matrice ARN. La transcription dans ces conditions permet d'obtenir au moins 50 transcrits par matrice. Selon l'invention, la région "séquence d'initiation I₁" (ou I₂) est présente sous forme ARN dans le produit de transcription qui va devenir cible à son tour pour la réaction d'amplification cyclique. La construction des oligonucléotides chimères rend possible la digestion spécifique (dans la région complémentaire de la séquence d'initiation, notamment) par la RNase H, d'une partie des ARN à amplifier ou à réamplifier après l'hybridation de l'oligonucléotide chimère, permettant ainsi la synthèse d'une séquence ADN matrice complémentaire de cette même séquence d'initiation. Cela permet d'obtenir la région d'initiation I sous forme d'un homoduplex DNA/DNA et d'initier la transcription par l'ARN polymérase ARN dépendante sur un brin matrice ADN, la transcription se poursuivant seulement à partir de la région complémentaire à la séquence sonde S sur une matrice ARN. La longueur de la séquence d'initiation sera comprise par exemple entre 4 et 18 nucléotides.

La transcription par l'ARN polymérase permet la synthèse de multiples transcrits. Ces transcrits comportent une séquence entièrement complémentaire de celle de l'acide nucléique de départ exception faite de leur extrémité 5' qui a été définie grâce à la coupure de l'ARN cible par la RNase H sur la région hybridée à la région d'initiation I₁ sur l'oligonucléotide chimère. Ainsi, si l'oligonucléotide chimère s'est hybridé complètement à l'extrémité 3' de l'acide nucléique de départ, et si la séquence d'initiation I₁ ne comporte que des bases strictement complémentaires de celles de cette cible, l'extrémité 5' des transcrits est complémentaire de l'extrémité 3' de l'acide nucléique de départ. Au contraire, si l'oligonucléotide chimère ne s'est pas hybridé strictement à l'extrémité 3' de la molécule de départ, les transcrits seront plus courts que cette molécule de départ. Dans tous les cas, l'extrémité 5' des néo-transcrits sera définie, puisque cette séquence sera obligatoirement homologue de celle des régions I₁ et S₁ de l'oligonucléotide chimère.

Selon un mode de réalisation particulier, la région d'initiation I₁ peut contenir dans sa partie 5' des nucléotides non appariés avec la molécule de départ. En conséquence, l'extrémité 5' des néo-transcrits contient une séquence non totalement complémentaire de celle de cette cible et que l'on peut choisir arbitrairement. Cette faculté de choix peut être mise à profit pour favoriser par exemple une augmentation du facteur de transcription, notamment en choisissant la séquence consensus d'initiation spécifique de la transcription pour le promoteur et l'enzyme utilisés. Par exemple, pour la T3 ARN polymérase, la T7 ARN polymérase et la K11 ARN polymérase, cette séquence sera préférentiellement la séquence GGGAGA ; pour la SP6 ARN polymérase, cette séquence sera préférentiellement la séquence GAAGGG ; pour la BA14 ARN polymérase, cette séquence sera préférentiellement la séquence GCGAGA. Le choix de cette partie de I₁ non complémentaire de la cible peut permettre aussi, par exemple, une stabilisation du transcrit ou l'apport d'une séquence particulière permettant la détection.

Les néo-transcrits, issus de la transcription sous le contrôle du promoteur présent dans le segment P₁, peuvent s'hybrider avec le second oligonucléotide chimère par l'intermédiaire de la séquence sonde (S₂), et par l'intermédiaire de tout ou partie de la séquence d'initiation (I₂). Ce second oligonucléotide chimère possède les mêmes caractéristiques que le premier oligonucléotide chimère promoteur-sonde décrit précédemment, mis à part le fait qu'il est capable d'hybridation non avec la séquence aval de la cible de départ, mais avec une séquence aval du complémentaire de la cible (ladite séquence aval du complémentaire étant le complémentaire de la séquence amont de la cible de départ). Préférentiellement, il porte la même séquence promoteur que le premier oligonucléotide chimère promoteur-sonde. A nouveau, la partie ARN de la région hétéroduplex ARN:ADN formée par hybridation des transcrits ARN avec tout ou partie de la séquence d'initiation de type ADN (I₂) est dégradée, notamment par digestion par la RNase H. Comme indiqué précédemment, la région d'initiation I₂ peut renfermer du côté 5' une séquence non hybridée avec les transcrits. La séquence sonde S₂ étant suffisament longue ou l'hybridation suffisament stable, les transcrits restent hybridés avec le second oligonucléotide chimère. Une ADN polymérase ADN dépendante appropriée permet alors la synthèse du brin anti-sens du promoteur par élongation des transcrits le long de l'oligonucléotide chimère. Préférentiellement, la même ADN polymérase est utilisée pour l'installation du deuxième brin des promoteurs des premier et second oligonucléotides chimères. La transcription avec l'ARN polymérase appropriée permet à nouveau la synthèse de multiples transcrits.

Ces transcrits portent à leur extrémité 3' les séquences complémentaires de I₁ et Si permettant l'hybridation avec le premier oligonucléotide chimère (P₁,I₁,S₁). Le brin ARN du nouvel hétéroduplex ADN:ARN formé, c'est à dire la région du transcrit complémentaire de la région I₁ est le substrat de la RNase H. L'élongation de l'extrémité 3' du transcrit, à l'aide d'une ADN polymérase, va alors former le brin anti-sens du promoteur P₁. Une ARN polymérase spécifique du promoteur P₁ permet ensuite la synthèse de multiples transcrits qui portent à leur extrémité 3' une séquence complémentaire des régions I₂ et S₂ du second oligonucléotide chimère, permettant l'hybridation avec celui-ci. Le brin ARN de ce nouvel hybride ADN:ARN peut être dégradé, dans sa région complémentaire de la région I₂, par la RNase H. Le cycle peut alors être réitéré

Le procédé de l'invention, grâce à la répétition de l'installation de l'oligonucléotide chimère sur une séquence d'acide ribonucléique, conduit à la synthèse de multiples transcrits, et permet d'amplifier exponentiellement le fragment d'acide nucléique cible initial

On peut constater aisément que pour que le procédé de l'invention puisse fonctionner avec une cible d'ADN, il est nécessaire que l'extrémité 3' de cet ADN soit définie. Autrement dit, dans ce cas, la séquence aval de la séquence-cible correspond à la séquence d'extrémité 3' de l'ADN de départ Cela peut être fait de façon connue, dans des opérations préalables à la mise en oeuvre du procédé de l'invention. Pour définir une extrémité sur un ADN double brin, on peut couper la cible avec des enzymes de restriction. Si l'ADN est simple brin, on peut hybrider, sur l'endroit à couper, des oligodésoxyribonucléotides, ce qui permet la coupure par des enzymes de restriction On peut également définir l'extrémité 3' en utilisant un bloqueur: sur un ADN simple brin, on peut hybrider deux oligonucléotides sur les régions qui doivent devenir les extrémités. L'oligonucléotide amont est soumis à une élongation enzymatique. Lorsque l'enzyme arrive à l'oligonucléotide aval, appelé oligonucléotide bloqueur, la réaction d'élongation est arrêtée. Cet arrêt peut être induit par différents facteurs ; en particulier, on peut utiliser une polymérase dépourvue d'activité de déplacement, et qui sera bloquée par un oligonucléotide constitué de bases naturelles. Si la polymérase a une activité de déplacement, l'oligonucléotide bloqueur peut comporter des modifications chimiques induisant le blocage (comme par exemple du psoralène), ou des modifications au niveau de la liaison internucléotidique (comme par exemple les liaisons phosphorothioate, H-phosphonate, alkyl phosphonate), ou encore au niveau du squelette comme par exemple les alpha-oligonucléotides (brevet Français n° 2 607 507) ou les PNA (Egholm *et al*., 1992. *J*. *Am. Chem. Soc*. 114 : 1895-1897). Une autre méthode consiste à utiliser une séquence d'initiation I comprenant un site de restriction présent sur la séquence cible, ledit site contenant des nucléotides modifiés dont la nature ne permet pas la coupure par l'enzyme de restriction. On obtient ainsi une extrémité 3' définie sur une cible ADN par clivage sélectif du brin cible.

Les exemples suivants illustrent l'invention. Sauf précision contraire, les méthodes relatives à la mise en oeuvre des exemples décrits ci-après sont conformes à leur description par Sambrook *et al*. (1989. Molecular Cloning : A Laboratory Manual, 2nd Edition, Cold Spring Harbor Laboratory, Cold Spring Harbor).

Les essais ont été effectués sur la séquence du gène TEM codant pour une β-lactamase induisant une résistance à l'ampicilline et présent, en particulier, dans le plasmide pBR322.

D'une façon générale, les réactions décrites ci-après sont réalisées dans un volume final de 20 µl de tampon : 40 mM Tris-HCI pH 8,1, 10 mM MgCl₂, 1 mM NTP, 1 mM dNTP, 5 mM DTT, 1 mM spermidine-HCl, 8% polyéthylène glycol, 0,01 % triton X100, 50 µg/ml albumine de sérum de boeuf; toutefois, certains de ces paramètres pourront être ajustés pour chacun des protocoles. Les enzymes utilisées sont la T7 ARN polymérase (Ozyme-Biolabs), la T3 ARN polymérase (Pharmacia), la SP6 ARN polymérase (Pharmacia), la Klenow sequencing grade (nom commercial ; Boehringer), la klenow exo (-) (USB), la transcriptase inverse Superscript II (nom commercial ; Gibco BRL), la Sequenase (nom commercial ; USB), la RNase H d'*E. coli* (Boerhinger).

NTP et dNTP désignent les nucléosides triphosphates.

En général, les conditions réactionnelles n'ont pas fait l'objet d'expériences préalables d'optimisation.

### Exemple 1 : Synthèse des oligonucléotides chimères ADN/ARN

Les oligonucléotides chimères T7(+)-A27-ARNA28 (SEQ ID N° 27) et T7(+)-A21-ARNA20 (SEQ ID N°26) sont préparés sur synthétiseur APPLIED BIOSYSTEMS 394 DNA/RNA. La synthèse se déroule de 3' en 5' en utilisant la chimie des phosphoramidites. La partie ARN est réalisée avec des ribonucléotides fournis par la Société APPLIED. Ils sont protégés en 2' par un groupement ter-butyldiméthylsilyl (t-BDMS), (Admf: réf401350, Gdmf: réf401351, Cibu : réf401352 et U: réf401353). La partie ARN se situant en 3', la synthèse des chimères démarre avec un support portant un ribonucléotide. Les supports de synthèse sont des colonnes ARN 1µmol 1000A, adénosine-WPS de chez BIOGENEX (réf 8310-1).

La partie ADN est réalisée avec des désoxyribonucléotides à déprotection rapide fournis par la société PHARMACIA (PAC dA : réf 27-1723-03, iPr-PAC dG : réf 27-1726-03, IBU dC : réf 27-1725-03). Le désoxyribonucléoside T est fourni par APPLIED BIOSYSTEMS (réf 400329).

Les deux cycles de synthèse utilisés sont conformes aux instructions du constructeur.

Une solution d'ammoniac dans l'éthanol anhydre est utilisée pour cliver le chimère du support. La solution est obtenue en faisant buller de l'ammoniac anhydre (ALDRICH réf 29, 499-3) dans de l'éthanol pur (MERCK réf 983) pendant 1 h.

La déprotection de l'oligonucléotide chimère, à l'exception des groupements hydroxyles en 2' de la partie ARN, est réalisée dans cette même solution ammoniacale, une nuit à température ambiante.

Après évaporation de la solution ammoniacale, à l'évaporateur rotatif, 50 équivalents de fluorure de tétrabutylammonium (TBAF 1,1M/THF, ALDRICH 21, 614-3), sont utilisés pour déprotéger les groupements hydroxyles en 2'. Après 24h de contact à température ambiante avec le TBAF, le chimère est séché à l'évaporateur rotatif puis repris dans 1 ml d'eau milli Q (marque de commerce) stérile. La solution aqueuse est lavée à l'acétate d'éthyle et dessalée sur colonne échangeuse d'ions dans les conditions suivantes :
Colonne : WATERS protein-pak DEAE 15HR
15µm, 1000A
AP-1 10*100 mm
Tampons : A=TEAB 0,1M(TEAB=bicarbonate de triéthylamine) B=TEAB 2M

Le produit est chargé sur la colonne avec 100% de tampon A pendant 20 min puis élué avec 100% de tampon B pendant 30 min avec un débit de 4 ml/min.

Le tampon B est réalisé en faisant buller du dioxyde de carbone (ALDRICH réf 29,510-8) dans un mélange triéthylamine (ALDRICH réf 23,962-3)/eau stérile.

Après dessalage, la molécule chimère est séchée à l'évaporateur rotatif puis reprise dans 1 ml d'eau stérile. Ensuite, le chimère est purifié par chromatographie en phase inverse dans les conditions suivantes.
Colonne préparative sur Ultrapore RPMC Beckman
dp 5µm, 10*250mm
N° de série n° 238770
Tampons : A=TEAA 0,1M B=TEAA 0,1M/CH₃CN 50%
Références : Triéthylammonium acétate (TEAA)2 M HPLC grade
APPLIED BIOSYSTEMS
Acétonitrile : BAKER HPLC gradient grade
Gradient : de 20 à 30 % de B en 10 min, puis de 30 à 70 % de B en 20 min avec un débit de 4,7 ml/min.

### Exemple 2 : Elongation d'une amorce ARN sur une matrice ADN.

Cet exemple a pour but de montrer qu'une amorce ARN peut servir de substrat à une ADN polymérase en vue de son élongation sur une matrice ADN. Pour ce faire, plusieurs oligonucléotides ont été utilisés. L'oligonucléotide T7(+)-A27-A28 (SEQ ID No 8) constitue la matrice ADN et porte de son extrémité 5' vers son extrémité 3', la séquence sens du promoteur T7, une séquence A27 et une séquence A28. L'oligonucléotide ARNA19 (SEQ ID No 6) et l'oligonucléotide A19(SEQ ID No 7) sont des amorces de nature ARN et ADN, respectivement, dont la séquence est complémentaire de la séquence sonde A28 de l'oligonucléotide T7(+)-A27-A28 (SEQ ID No 8). Ces deux oligonucléotides sont radiomarqués au ³² P à leur extrémité 5'-OH, par la polynucléotide kinase .

L'oligonucléotide T7(+)-A27-A28 (SEQ ID No 8) et l'oligonucléotide ARN19 (SEQ ID No 6) ou A19 (SEQ ID No 7) sont incubés pendant 5 minutes à 65°C à la concentration de 5.10¹⁰ copies/ µl chacun dans un volume final de 20 µl de milieu réactionnel tel que décrit précédemment, ajusté à 20 mM MgCl₂ et en absence de NTP. Les tubes sont ensuite placés 10 minutes à température ambiante afin de permettre l'hybridation des oligonucléotides, puis préincubés environ 1 minute à 37°C avant 1' addition de 13 unités de Sequenase, ou de 10 unités de klenow, ou de 130 unités de Superscript II transcriptase inverse. On effectue en parallèle des contrôles réactionnels sans enzyme et/ou sans matrice. Après incubation pendant 1 heure à 37°C, on arrête les réactions par refroidissement sur de la glace. Une partie de chaque échantillon (10 µl) est mélangée avec 10µl de "bleu formamide" (90% formamide, 0,02 xylène cyanole, 0,02% bleu de bromophénol, 25 mM EDTA) puis analysée par électrophorèse sur gel 15% polyacrylamide - 7M urée en présence d'un marqueur de poids moléculaire formé par un mélange d'oligodésoxyribonucléotides de 60, 55, 50, 45, 40, 35, 30 et 25 nucléotides. Après séchage, le gel est autoradiographié sur film X-Ray.

Quelle que soit l'enzyme utilisée, on observe une élongation équivalente à partir d'une amorce ADN ou d'une amorce ARN. On peut toutefois noter, dans le cas de l'élongation par la Sequenase, la présence de produits d'extension plus longs que ceux attendus (50 nucléotides).

### Exemple 3 : Transcription par la T7 ARN polymérase, la T3 ARN polymérase et la SP6 ARN polymérase sur matrice simple ou double brin de nature entièrement ADN ou ARN à partir de la position +18. Effet de l'ADN exogène.

Les activités de la T3 ARN polymérase, de la SP6 ARN polymérase et de la T7 ARN polymérase ont été testées sur différents types de matrices. Pour cela, une série d'oligonucléotides a été construite (leur composition est indiquée de 5' vers 3', avec l'extrémité 5' à gauche) :
* pour l'analyse de la transcription par la T3 ARN polymérase :
   - oligonucléotide T3(-)-A18-A19 (SEQ ID N° 16) : la séquence anti-sens du promoteur T3, une séquence A18 de nature ADN et une séquence A19 de nature ADN,
   - oligonucléotide T3(+)-A27-A28 (SEQ ID N° 12 ) : la séquence sens du promoteur T3, une séquence d'initiation A27 de nature ADN et une séquence sonde A28 de nature ADN,
   - oligonucléotide T3(+) (SEQ ID N° 13) : la séquence sens du promoteur T3,
   - oligonucléotide chimère T3(-)-A18-ARNA19 (SEQ ID N°17) : la séquence anti-sens du promoteur T3, une séquence A18 de nature ADN et une séquence A19 de nature ARN,
* pour l'analyse de la transcription par la SP6 ARN polymérase :
   - oligonucléotide SP6(-)-A18-A19 (SEQ ID N° 14) : la séquence anti-sens du promoteur SP6, une séquence A18 de nature ADN et une séquence A19 de nature ADN,
   - oligonucléotide SP6(+)-A27-A28 (SEQ ID N°10) : la séquence sens du promoteur SP6, une séquence d'initiation A27 de nature ADN et une séquence sonde A28 de nature ADN,
   - oligonucléotide SP6(+) (SEQ ID N° 11) : la séquence sens du promoteur SP6,
   - oligonucléotide chimère SP6(-)-A18-ARNA19 (SEQ ID N° 19) : la séquence anti-sens du promoteur SP6, une séquence A18 de nature ADN et une séquence A19 de nature ARN,
* pour l'analyse de la transcription par la T7 ARN polymérase :
   - oligonucléotide T7(-)-A18-A19 (SEQ ID N° 15): la séquence anti-sens du promoteur T7, une séquence A18 de nature ADN et une séquence A19 de nature ADN,
   - oligonucléotide T7(+)-A27-A28 (SEQ ID N° 8) : la séquence sens du promoteur T7, une séquence d'initiation A27 de nature ADN et une séquence sonde A28 de nature ADN,
   - oligonucléotide T7(+) (SEQ ID N° 9) : la séquence sens du promoteur T7,
   - oligonucléotide chimère T7(-)-A18-ARNA19 (SEQ ID N° 18): la séquence anti-sens du promoteur T7, une séquence A18 de nature ADN et une séquence A19 de nature ARN.

Pour l'étude de l'activité de la T3 ARN polymérase sur matrice ADN, l'oligonucléotide matrice T3(-)-A18-A19 (SEQ ID No 16) est hybridé soit avec l'oligonucléotide T3(+)-A27-A28 (SEQ ID No 12), pour l'étude de la transcription sur une matrice double brin ; soit avec l'oligonucléotide T3(+) (SEQ ID No 13), pour l'étude de la transcription sur une matrice simple brin ; soit l'oligonucléotide T3(-)-A18-A19 (SEQ ID No 16) est testé seul, pour l'étude de la transcription avec un promoteur simple brin et sur une matrice simple brin.

Pour l'étude de l'activité de la T3 ARN polymérase sur matrice de nature ADN de la position +1 à la position +17 et de nature ARN à partir de la position +18 (positions par rapport au site d'initiation de la transcription), l'oligonucléotide matrice T3(-)-A18-ARNA19 (SEQ ID No 17) est hybridé avec les mêmes oligonucléotides non matrice que dans le cas précédent.

De même, pour l'étude de l'activité de la SP6 ARN polymérase sur matrice ADN, l'oligonucléotide matrice SP6(-)-A18-A19 (SEQ ID No 14) est hybridé soit avec l'oligonucléotide SP6(+)-A27-A28 (SEQ ID No 10), pour l'étude de la transcription sur matrice double brin ; soit avec l'oligonucléotide SP6(+) (SEQ ID No 11), pour l'étude de la transcription sur la matrice simple brin ; soit l'oligonucléotide SP6(-)-A18-A19 (SEQ ID No 14) est testé seul, pour l'étude de la transcription avec un promoteur simple brin et sur une matrice simple brin.

Pour l'étude de l'activité de la SP6 ARN polymérase sur matrice de nature ADN de la position +1 à la position +17 et de nature ARN à partir de la position +18, l'oligonucléotide matrice SP6(-)-A18-ARNA19 (SEQ ID No 19) est hybridé avec les mêmes oligonucléotides non matrice que dans le cas précédent. Enfin, pour l'étude de l'activité de la T7 ARN polymérase sur matrice ADN, l'oligonucléotide matrice T7(-)-A18-A19 (SEQ ID No 15) est hybridé soit avec l'oligonucléotide T7(+)-A27-A28 (SEQ ID No 8), pour l'étude de la transcription sur matrice double brin ; soit avec l'oligonucléotide T7(+) (SEQ ID No 9), pour l'étude de la transcription sur la matrice simple brin ; soit l'oligonucléotide T7(-)-A18-A19 (SEQ ID No 15) est testé seul, pour l'étude de la transcription avec un promoteur simple brin et sur une matrice simple brin.

Pour l'étude de l'activité T7 ARN polymérase sur matrice de nature ADN de la position +1 à la position +17 et de nature ARN à partir de la position +18, l'oligonucléotide matrice T7(-)-A18-ARNA19 (SEQ ID No 18) est hybridé avec les mêmes oligonucléotides non matrice que dans le cas précédent.

En outre, les essais utilisant la T7 ARN polymérase ont également été effectués en présence de 10 ng ou de 100 ng d'ADN de sperme de saumon, afin de tester l'effet de la présence d'ADN exogène sur la transcription.

Dans ces tests, les oligonucléotides sont à la concentration de 5.10⁹ copies/µl chacun. Les réactions sont effectuées dans un volume final de 20µl de milieu réactionnel décrit précédemment, en l'absence de dNTP, et en prenant soin d'ajuster les conditions en fonction de la polymérase testée comme indiqué ci-après : 10 mM MgCl₂ et 1 mM CaCl₂ pour la T3 ARN polymérase ; 4 mM MgCl₂ pour la SP6 ARN polymérase ; et 6 mM MgCl₂ pour la T7 ARN polymérase. Les échantillons sont dénaturés par chauffage à 65°C durant 5 minutes puis descendus à 37°C en 10 min. La réaction de transcription est alors amorcée par l'addition de 50 unités de polymérase, et après incubation pendant deux heures à 37°C, la réaction est stoppée sur de la glace. Les échantillons de 10 µl sont mélangés avec 10 µl de "bleu formamide" et analysés par électrophorèse sur gel 20% polyacrylamide - 7M urée, suivie d'un électrotransfert sur Hybond N (nom commercial, Amersham), hybridation avec l'oligonucléotide A19 (SEQ ID N° 7) marqué à la peroxydase et révélation par un substrat colorimétrique, la diaminobenzidine.

On a trouvé que quelle que soit l'enzyme utilisée, les résultats de la transcription sur matrice ADN sont équivalents, que la matrice soit sous forme double ou simple brin. Par contre, les conditions expérimentales telles que décrites ne permettent pas la transcription à partir d'un promoteur ADN simple brin sur une matrice ADN simple brin. De même, aucune transcription n'est observée à partir d'un promoteur ADN simple brin sur matrice ARN simple brin pour aucune des trois enzymes.

L'étude de la transcription par la T3 ARN polymérase ou par la T7 ARN polymérase, en présence d'un promoteur ADN double brin sur matrice ARN à partir de la position +18 [oligonucléotides T3 ou T7 (-)-A18- ARNA19], montre que le taux de transcription obtenu avec cette matrice sous sa forme double brin est plus faible que celui obtenu sur une matrice ADN (simple ou double brin); par contre, le taux obtenu avec cette même matrice sous sa forme simple brin est aussi élevé que celui obtenu sur une matrice ADN. Les résultats observés dans le cas de la SP6 ARN polymérase montrent que le taux de transcription sur matrice ARN simple ou double brin est plus faible que celui sur matrice ADN.

L'étude de l'effet de la présence d'ADN de saumon sur la transcription par la T7 ARN polymérase montre que le taux de cette transcription n'est pas affecté par la présence d'ADN exogène.

Le facteur de transcription étant défini comme le nombre de transcrits obtenus par matrice, on peut estimer ce facteur à environ 10 pour la transcription sur matrice double brin et ARN à partir de +18 pour la T3 ARN polymérase, à environ 50 pour la transcription sur matrice simple brin et ARN à partir de +18 pour cette même enzyme. Pour la SP6 ARN polymérase, le facteur de transcription sur matrice double brin ou simple brin et ARN à partir de +18 peut être évalué à environ 10, pour la T7 ARN polymérase, le facteur de transcription sur matrice double brin et ARN à partir de +18 peut être évalué à environ 50, et à environ 100 sur simple brin.

### Exemple 4 : Transcription sur matrice ARN à partir de la position +1 par la T3 ARN polymérase. Transcription sur matrice simple ou double brin.

L'activité de la T3 ARN polymérase a été testée sur une matrice entièrement ARN. Pour cela, un oligonucléotide chimère T3(-)-ARNA18-ARNA19 (SEQ ID N° 20) a été construit qui renferme de son extrémité 5' vers son extrémité 3': la séquence ADN anti-sens du promoteur de la T3 ARN polymérase, une séquence ARNA18 de nature ARN et une séquence ARNA19 de nature ARN. Cet oligonucléotide est hybridé soit avec l'oligonucléotide T3(+) (SEQ ID N° 13) pour tester l'activité sur matrice ARN simple brin, soit avec l'oligonucléotide T3(+)-A27-A28 (SEQ ID N° 12), pour tester l'activité sur matrice double brin. Les réactions sont effectuées dans 20 µl de milieu réactionnel décrit précédemment, en absence de dNTP, ajusté à 10 mM MgCl₂ et chacun des oligonucléotides étant présent à la concentration de 5.10⁹ copies/µl. Les réactions ont en outre été testées en présence de 10% glycérol ou de 3% glycérol. Après incubation pendant 5 minutes à 65°C, puis refroidissement à 37°C en 10 min, la réaction est amorcée par addition de 50 unités de T3 ARN polymérase. On laisse incuber pendant 2 heures à 37°C, puis les réactions sont arrêtées sur de la glace. Une fraction aliquote (10 µl) des produits de la réaction sur matrice double brin est soumise à la digestion par la DNase I. L'analyse des réactions est effectuée par électrophorèse d'une partie aliquote de 10µl sur gel 20% polyacrylamide-7M urée, électrotransfert sur Hybond N, hybridation avec l'oligonucléotide A19 ( SEQ ID N° 7) marqué par la peroxydase et révélation par un substrat chemiluminescent, le luminol.

Le même essai-témoin conduit dans l'exemple N°3 a été conduit ici et permet de montrer que le procédé d'analyse utilisant comme substrat le luminol est suffisament sensible pour permettre la mise en évidence des 5X10¹⁰ copies d'oligonucléotide non matrice T3 (+)-A27-A28 (SEQ ID N° 12) présents sur les blots dans le cas des réactions sur matrice double brin. Cependant, le transcrit obtenu est de taille inférieure. En outre, la digestion par la DNase I permet d'éliminer le bruit de fond dû à cette hybridation. Les résultats montrent que l'on obtient une transcription sur matrice ARN à partir du +1 aussi bien avec la forme double que simple brin, mais que le taux de cette transcription est faible : on obtient environ un transcrit pour 10 matrices.

### Exemple 5 : Réitération du processus d'installation du promoteur et transcription sur matrice ARN.

Afin de démontrer la possibilité d'installer un promoteur sur un ARN, d'obtenir des transcrits complémentaires, de réitérer l'installation du promoteur sur ces transcrits et d'obtenir des transcrits complémentaires des transcrits de la première réaction, un oligonucléotide cible ARNA20-ARNA19 (SEQ ID N° 25) de nature entièrement ARN a été construit.

Cet oligonucléotide a été mélangé aux oligonucléotides T7(+)-A27-A28 (SEQ ID N° 8) et T7(+)-A21-A20 (SEQ ID N° 25) à la concentration de 5.10¹⁰ copies/µl chacun dans 20 µl de milieu réactionnel décrit précédemment. Après dénaturation pendant 5 minutes à 65°C, les tubes sont incubés pendant 10 minutes à température ambiante, puis 1 minute à 37°C avant l'addition de 10 unités de klenow. On fait incuber à 37°C pendant 30 minutes. L'enzyme est dénaturée pendant 10 minutes à 65°C. On effectue une nouvelle incubation pendant 10 minutes à température ambiante afin de permettre l'hybridation des oligonucléotides. Après 1 minute de préincubation à 37°C, on ajoute 50 unités de T7 ARN polymérase. On laisse incuber durant 1 heure à 37°C. Certaines réactions sont arrêtées à ce stade.

Les réactions sur lesquelles on veut effectuer une deuxième étape de transcription sont à nouveau chauffées à 65°C pendant 10 minutes, de façon à dénaturer la T7 ARN polymérase. Après incubation à température ambiante durant 10 minutes, on ajoute à nouveau 10 unités de klenow et met en incubation à 37°C pendant 30 minutes. Après un nouveau cycle de dénaturation de la klenow (65°C, 10 minutes ; température ambiante, 10 minutes ; préincubation à 37°C), on ajoute 50 unités de T7 ARN polymérase et met en incubation 60 minutes à 37°C. Les réactions sont stoppées dans la glace. Un cinquième du produit de la réaction (représentant 2X10¹¹ copies de chacun des oligonucléotides initialement présents) est mélangé avec un volume égal de "bleu formamide" et est analysé sur gel 20% polyacrylamide-7M urée. Différents échantillons de la même réaction sont analysés en parallèle. Après électrotransfert sur Hybond N, les blots sont hybridés soit avec l'oligonucléotide A20 (SEQ ID N°22), soit avec l'oligonucléotide A26 (SEQ ID N°23) marqués à la digoxigénine, révélés par le système de détection de Boehringer mettant en jeu une réaction immunoenzymatique avec un anticorps anti-digoxigénine et un substrat luminescent , puis autoradiographie par films X-Ray.

Lors de la première étape de transcription, l'oligonucléotide non matrice T7(+)-A27-A28 (SEQ ID N°8) peut s'hybrider sur la cible ARNA20-ARNA19 (SEQ ID N°21) car la séquence A28 est complémentaire de l'oligonucléotide A19. Le second oligonucléotide T7(+)-A21-A20 (SEQ ID N°25) qui est présent dans la réaction ne joue aucun rôle. La klenow étend la cible ARN en utilisant l'oligonucléotide T7(+)-A27-A28 (SEQ ID N° 8) comme matrice. La T7 ARN polymérase pourra alors reconnaître le promoteur dont le brin anti-sens a été formé et synthétiser un transcrit de séquence complémentaire à la séquence de la cible ARN. La sonde oligonucléotide A20 (SEQ ID N°22) permet de révéler de manière spécifique ces produits de réaction.

Lors de la seconde étape de transcription, l'oligonucléotide T7(+)-A21-A20 (SEQ ID N°25) s'hybride sur l'extrémité 3' des produits de la première réaction. La klenow effectue l'élongation de l'extrémité 3' de ces ARN en utilisant l'oligonucléotide T7(+)-A21-A20 (SEQ ID N°25) comme matrice et forme ainsi le brin anti-sens du promoteur. La T7 ARN polymérase synthétise alors les transcrits complémentaires des transcrits de la première étape. La sonde oligonucléotide A26 (SEQ ID N°23) permet de révéler ces néo-transcrits. Le promoteur T7(+)-A21-A20 est également mis en évidence par la sonde A26, néanmoins, les transcrits attendus ont une taille de 65 bases tandis que cet oligonucléotide ne comporte que 50 nucléotides.

Les résultats obtenus montrent qu'après une première étape de transcription, on obtient des transcrits de taille attendue et hybridant à la sonde A20. Après la seconde étape de transcription, une bande correspondant à un poids moléculaire d'environ 80 bases apparaît alors que la bande correspondant aux transcrits de la première étape diminue d'intensité. Cela indique que l'on a bien extension des transcrits de la première étape.

Les résultats de l'hybridation avec la sonde A26 montrent une bande de la taille de l'oligonucléotide T7(+)-A21-A20 (SEQ ID N° 25) présente même en absence d'enzyme. La première étape de transcription ne permet pas l'apparition d'autres bandes s'hybridant avec A26. Par contre, la seconde étape de transcription fait apparaître des bandes de plus haut poids moléculaire, dont la bande majoritaire présente une migration électrophorétique correspondant à un produit de taille attendue.

Ces expériences montrent donc la possibilité de réitérer l'installation d'un promoteur et la transcription à partir d'une matrice formée par un ARN néo-transcrit.

### Exemple 6 : Installation de l'oligonucléotide chimère sur matrice ARN et réaction de transcription

L'ARN cible correspond à la séquence ARNA20-ARNA19-ARNA18 (SEQ ID28). L'oligonucléotide chimère utilisé est T7(+)-A27-ARNA28 (SEQ ID27). Cet oligonucléotide chimère renferme, de 5' vers 3':
- un premier segment nucléique T7(+), de nature ADN, correspondant à la séquence sens d'un promoteur pour la T7 ARN polymérase,
- un second segment nucléique A27, de nature ADN, correspondant à une séquence d'initiation,
- et un troisième segment nucléique ARNA28, de nature ARN et correspondant à une séquence sonde.

Il est utile de mentionner que les segments A27 et ARNA28 de l'oligonucléotide chimère sont complémentaires des séquences ARNA18 et ARNA19 de l'ARN cible, respectivement. Par ailleurs, le blocage de l'extrémité 3' de l'oligonucléotide est obtenu par la présence des deux ribonucléotides 3'-terminaux qui ne peuvent pas s'apparier avec la cible.

Un témoin réactionnel est réalisé avec la cible de nature ARN ARNA20-ARNA19 (SEQ ID N° 21) qui correspond à la séquence de la cible ARNA20-ARNA19-ARNA18 (SEQ IDN° 28) privée de la séquence ARNA18, substrat de la RNase H dans l'hybride oligonucléotide chimère T7(+)-A27-ARNA28/cible ARNA20-ARNA19-ARNA18.

La réaction est effectuée dans le milieu réactionnel précédemment décrit, en présence de 5.10⁹ copies/µl d'oligonucléotide chimère et de 5.10⁹ copies/µl d'ARN cible, dans un volume final de 20 µl. Pour permettre une bonne hybridation au départ, le mélange est chauffé pendant 5 minutes à 65°C, puis progressivement refroidi à 37°C en 10 minutes. Après une brève préincubation à 37°C, on ajoute 1 unité de RNase H et on maintient à 37°C pendant 30 minutes. La RNase H est ensuite dénaturée par incubation à 65°C pendant 10 minutes. Après refroidissement progressif à température ambiante pendant 10 minutes, 10 unités de Klenow exo(-) sont ajoutées et on effectue une incubation pendant 30 minutes à 37°C. L'enzyme Klenow exo(-) est ensuite dénaturée par la chaleur et 50 unités de T7 ARN polymérase sont ajoutées. On fait incuber pendant 60 minutes à 37°C, puis on arrête les réactions par refroidissement sur de la glace. Des contrôles réactionnels sont effectués : réactions sans aucune enzyme, réactions sans T7 ARN polymérase, sans RNase H ou sans Klenow exo(-). La moitié du volume réactionnel des échantillons est analysée par électrophorèse sur gel de polyacrylamide 20% - 7M urée après mélange avec du "bleu formamide", électrotransfert sur Hybond N, hybridation à l'oligonucléotide A19 (SEQ ID N°7) marqué à la peroxydase et révélation par le substrat colorimétrique (diaminobenzidine).

Les résultats montrent que l'on obtient les transcrits attendus lorsque l'on utilise indifféremment la cible ARNA20-ARNA19 (SEQ ID N°21) ou la cible ARNA20-ARNA19-ARNA18 (SEQ ID N°28) dans les réactions mettant en jeu les trois enzymes. Aucune des réactions réalisées en l'absence de toutes les enzymes ou d'une des polymérases ne permet d'obtenir de transcrits. Les réactions réalisées sans RNase H ne permettent pas de produire des transcrits à partir de la matrice ARNA20-ARNA19-ARNA18 (SEQ ID N°28) tandis que les transcrits attendus sont obtenus lorsque l'on utilise la matrice ARNA20-ARNA19 (SEQ ID N°21).

De façon analogue, on a pu mettre en évidence la production de transcrits en ajoutant toutes les enzymes dès le début (après la préincubation à 37°C mentionnée ci-dessus) et en opérant ensuite à la température unique de 37°C.

### Exemple 7 :

Les cibles sont des monobrins d'ARN correspondant aux séquences SEQ ID N°33 et SEQ ID N°34, qui sont complémentaires (sens et antisens) l'un de l'autre. Ces brins d'ARN sont obtenus par transcription *in vitro* de fragments d'ADN, obtenus par PCR, qui renferment la séquence promotrice spécifiquement reconnue par la T7 ARN polymérase. Après transcription, les ARN sont purifiés sur gel d'acrylamide dénaturant.

Les oligonucléotides chimères utilisés, SEQ ID N°31 et SEQ ID N°32, renferment de 5' vers 3':
- un premier segment nucléique T7(+), de nature ADN, correspondant à la séquence sens d'un promoteur pour la T7 ARN polymérase,
- un second segment nucléique, de nature ADN, qui correspond à une séquence d'initiation et qui est complémentaire de l'extrémité 3' de l'ARN cible,
- et un troisième segment nucléique, de nature ARN, complémentaire de l'ARN cible.

L'oligonucléotide chimère SEQ ID N°31 est partiellement complémentaire de la cible SEQ ID N°33, et l'oligonucléotide chimère SEQ ID N° 32 est partiellement complémentaire de la cible SEQ ID N°34.

L'extrémité 3' de ces oligonucléotides chimères est bloquée par la présence d'un ou deux ribonucléotides 3'-terminaux (un seul ribonucléotide pour SEQ ID N°31) qui ne peuvent pas s'apparier avec la cible correspondante.

Des essais séparés ont été conduits en utilisant l'oligonucléotide SEQ ID N°31 et l'oligonucléotide SEQ ID N°32 comme amorces, et l'oligonucléotide SEQ ID N°33 et l'oligonucléotide SEQ ID N°34, respectivement, comme cibles.

5.10¹⁰ copies/ml d'oligonucléotide chimère et 5.10⁹ copies/ml d'ARN cible sont mélangés dans un volume final de 20 µl dans le milieu réactionnel tel que décrit dans l'exemple 6, auquel sont ajoutés 100 mM de glutamate de potassium. Le mélange est chauffé pendant 5 minutes à 65°C, puis refroidi à 37°C pendant 10 minutes. Les enzymes sont ajoutées en une seule étape sous la forme d'un mélange de 1 unité de RNase H thermostable "Hybridase" (Epicentre Technologies), 5 unités de klenow, et 250 unités de T7 ARN polymérase. Après incubation à 37°C pendant 60 minutes, les réactions sont stoppées par refroidissement sur de la glace. En parallèle, des contrôles réactionnels sont effectués dans des conditions identiques : soit sans aucune enzyme, soit sans ARN cible. La moitié du volume réactionnel est analysée par électrophorèse sur gel de polyacrylamide 15% - 7M urée après mélange avec du "bleu formamide", par electrotransfert sur Hybond N, et par hybridation avec l'oligonucléotide A21 (SEQ ID N°35) ou avec l'oligonucléotide A27 (SEQ ID N°36), qui sont, respectivement, spécifiques des transcrits issus de la cible ARN SEQ ID N°33 ou de la cible ARN SEQ ID N°34. Les oligonucléotides utilisés comme sondes de détection sont marqués à la peroxydase et révélés par le substrat colorimétrique DAB (diaminobenzidine).

Les résultats montrent que l'on obtient les transcrits attendus lorsque l'on utilise indifféremment la cible ARN sens ou antisens avec l'oligonucléotide chimère correspondant. Aucune des réactions réalisées en l'absence d'enzymes ou de cible ARN ne permet d'obtenir de transcrit.

## Revendications

1. Oligonucléotide chimère pouvant être utilisé dans un procédé d'obtention de transcrits et/ou d'amplification d'une séquence-cible d'un acide nucléique, ladite séquence-cible comprenant, à son extrémité 3', une séquence aval, ledit oligonucléotide comprenant successivement, de son extrémité 5' vers son extrémité 3':
- un premier segment oligonucléotidique, de type ADN, comprenant une séquence sens d'un promoteur d'une ARN polymérase,
- un second segment oligonucléotidique, de type ADN, comprenant le site d'initiation de la transcription pour ledit promoteur, au moins la région 3' dudit second segment étant capable de s'hybrider avec au moins une partie de ladite séquence aval,
- et un troisième segment oligonucléotidique, de type ARN, dont au moins la région comprenant l'extrémité 5' dudit troisième segment est capable de s'hybrider avec une partie de la séquence-cible contiguë à la partie de ladite séquence aval qui est capable d'hybridation avec ledit second segment, ledit troisième segment étant bloqué en 3'.

2. Oligonucléotide selon la revendication 1, caractérisé par le fait que ledit second segment contient de 2 à 30 nucléotides, et en particulier de 4 à 20 nucléotides.

3. Oligonucléotide selon l'une quelconque des revendications précédentes, caractérisé par le fait que ledit troisième segment contient de 2 à 30 nucléotides, et en particulier de 4 à 20 nucléotides.

4. Ensemble d'oligonucléotides pour l'obtention de transcrits, et/ou pour l'amplification cyclique, avec obtention de transcrits, d'une séquence-cible d'un acide nucléique et/ou d'une séquence complémentaire de ladite séquence-cible, ledit ensemble comprenant :
- un premier oligonucléotide chimère, tel que défini dans l'une quelconque des revendications précédentes, capable de s'hybrider avec une région aval de la séquence-cible,
- et un second oligonucléotide chimère, tel que défini dans l'une quelconque des revendications précédentes, capable de s'hybrider avec une région aval d'une séquence nucléotidique complémentaire de ladite séquence-cible.

5. Procédé d'obtention de transcrits, ou d'amplification cyclique avec obtention de transcrits, d'une séquence-cible d'un acide nucléique et/ou d'une séquence complémentaire de ladite séquence-cible, ladite séquence-cible comprenant à son extrémité 5' une séquence amont et à son extrémité 3' une séquence aval, non chevauchantes, ledit procédé comprenant, dans des conditions permettant l'hybridation et le fonctionnement des activités enzymatiques présentes, la mise en contact d'un échantillon contenant ou susceptible de contenir ledit acide nucléique avec :
a) un premier oligonucléotide chimère tel que défini dans l'une quelconque des revendications 1 à 3, capable de s'hybrider avec la séquence aval de la séquence-cible,
b) éventuellement un second oligonucléotide chimère, tel que défini dans l'une quelconque des revendications 1 à 3, capable de s'hybrider avec une région aval d'une séquence complémentaire de ladite séquence-cible, ladite région aval étant complémentaire de ladite séquence amont,
c) et un système enzymatique contenant une activité ADN-polymérase, une activité ARN-polymérase capable de fonctionner avec ledit promoteur et une activité capable de dégrader spécifiquement la région de la cible ou du complémentaire de la cible qui est capable d'appariement avec au moins une partie du deuxième segment du premier ou du deuxième oligonucléotide chimère, respectivement,
et l'incubation pendant un temps suffisant du mélange obtenu.

6. Procédé selon la revendication précédente, caractérisé par le fait que ladite activité de dégradation spécifique de la cible est une activité de RNase H.

## Patentansprüche

1. Chimäres Oligonucleotid, das in einem Verfahren zur Herstellung von Transkripten und/oder der Amplifikation einer Nucleinsäure-Zielsequenz verwendet werden kann, wobei die Zielsequenz an ihrem 3'-Ende eine stromab gelegene Sequenz umfasst, wobei das Oligonucleotid aufeinanderfolgend vom 5'-Ende zum 3'-Ende umfasst:
- ein erstes Oligonucleotidsegment vom DNS-Typ, umfassend eine sens-Sequenz eines Promoters einer RNS-Polymerase,
- ein zweites Oligonucleotidsegment vom DNS-Typ, umfassend die Transkriptions-Initiationsstelle für den Promoter, wobei mindestens der Bereich 3' des zweiten Segments mit mindestens einem Teil der stromab gelegenen Sequenz hybridisieren kann,
- ein drittes Oligonucleotidsegment vom DNS-Typ, wobei mindestens ein Bereich, umfassend das 5'-Ende des dritten Segments, mit einem Teil der Zielsequenz hybridisieren kann, benachbart dem Teil der stromab gelegenen Sequenz, die mit dem zweiten Segment hybridisieren kann, wobei das dritte Segment 3' blockiert ist.

2. Oligonucleotid gemäß Anspruch 1, dadurch gekennzeichnet, dass das zweite Segment 2 bis 30 Nucleotide, und insbesondere 4 bis 20 Nucleotide enthält.

3. Oligonucleotid gemäß einem der vorstehenden Ansprüche, dadurch gekennzeichnet, dass das dritte Segment 2 bis 30 Nucleotide, und insbesondere 4 bis 20 Nucleotide enthält.

4. Oligonucleotid-Satz zum Erhalten von Transkripten und/oder für die zyklische Amplifikation mit Erhalt von Transkripten einer Nucleinsäure-Zielsequenz und/oder einer zur Zielsequenz komplementären Sequenz, wobei der Satz umfasst:
- ein erstes chimäres Oligonucleotid, definiert gemäß einem der vorstehenden Ansprüche, welches mit einem stromab gelegenen Bereich der Zielsequenz hybridisieren kann,
- und ein zweites chimäres Oligonucleotid, definiert gemäß einem der vorstehenden Ansprüche, welches mit einem stromab gelegenen Bereich einer zur Zielsequenz komplementären Nucleotidsequenz hybridisieren kann.

5. Verfahren zum Erhalt von Transkripten oder der zyklischen Amplifikation unter Erhalt von Transkripten einer Nucleinsäure-Zielsequenz und/oder einer zur besagten Zielsequenz komplementären Sequenz, wobei die Zielsequenz an ihrem 5'-Ende eine stromauf und an ihrem 3'-Ende eine stromab gelegene Sequenz, nicht überschneidend, umfasst, wobei das Verfahren umfasst, unter Bedingungen, welche die Hybridisierung und ein Funktionieren der vorhandenen Enzymaktivitäten gestatten, das In-Kontakt-Bringen eines Puffers, der die Nucleinsäure enthält oder enthalten kann, mit:
a) einem ersten chimären Oligonucleotid, wie in einem der Ansprüche 1 bis 3 definiert, welches mit der stromab gelegene Sequenz der Zielsequenz hybridisieren kann,
b) eventuell einem zweiten chimären Oligonucleotid, wie definiert in einem der Ansprüche 1 bis 3, welches mit einem stromab gelegenen Bereich einer zur Zielsequenz komplementären Sequenz hybridisieren kann, wobei der stromab gelegene Bereich zu der stromauf gelegenen Sequenz komplementär ist,
c) und einem Enzymsystem, welches eine DNS-Polymerase-Aktivität, eine RNS-Polymerase-Aktivität, die mit dem Promoter funktionieren kann, und eine Aktivität umfasst, die spezifisch den Bereich des Ziels oder den zum Ziel komplementären Bereich abdauen kann, der mit mindestens einem Teil des zweiten Segments des ersten oder des zweiten chimären Oligonucleotids jeweils paaren kann, und Inkubation des erhaltenen Gemisches für ausreichende Zeit.

6. Verfahren gemäß dem vorstehenden Anspruch, dadurch gekennzeichnet, dass die Aktivität des spezifischen Abdauens des Ziels eine RNase H-Aktivität ist.

## Claims

1. Chimeric oligonucleotide which may be used in a method for prcducing transcripts and/or amplifying a target sequence of a nucleic acid, said target sequence comprising, at its 3' end, a downstream sequence, the said oligonucleotide comprising successively, from its 5' end to its 3' end:
- a first oligonucleotide segment, of the DNA type, comprising a sense sequence of a promoter of an RNA polymerase,
- a second oligcnucleotide segment, of the DNA type, comprising the site of initiation of transcription for the said promoter, at least the 3' region of the said second segment being capable of hybridizing with at least part of the said downstream sequence,
- and a third oligonucleotide segment, of the RNA type, of which at least the region comprising the 5' end of the said third segment is capable of hybridizing with part of the target sequence contiguous to the part of the said downstream sequence which is capable of hybridizing with the said second segment, the said third segment being blocked in 3'.

2. Oligonucleotide according to Claim 1, characterized in that the said second segment contains from 2 to 30 nucleotides, and in particular from 4 to 20 nucleotides.

3. Oligonucleotide according to any one of the preceding claims, characterized in that the said third segment contains from 2 to 30 nucleotides, and in particular from 4 to 20 nucleotides.

4. Set of oligonucleotides for the production of transcripts, and/or for the cyclic amplification, with the production of transcripts, of a target sequence of a nucleic acid and/or of a sequence complementary to the said target sequence, the said set comprising:
- a first chimeric oligonucleotide, as defined in any one of the preceding claims, capable of hybridizing with a downstream region of the target sequence,
- and a second chimeric oligonucleotide, as defined in any one of the preceding claims, capable of hybridizing with a downstream region of a nucleotide sequence complementary to the said target sequence.

5. Method for the production of transcripts, or for the cyclic amplification, with the production of transcripts, of a target sequence of a nucleic acid and/or of a sequence complementary to the said target sequence, the said target sequence comprising at its 5' end an upstream sequence and at its 3' end a downstream sequence, which are non-overlapping, the said method comprising, under conditions allowing the hybridization and the functioning of the enzymatic activities present, the bringing of a sample, containing or capable of containing the said nucleic acid, into contact with:
a) a first chimeric oligonucleotide as defined in any one of Claims 1 to 3, capable of hybridizing with the downstream sequence of the target sequence,
b) optionally a second chimeric oligonucleotide, as defined in any one of Claims 1 to 3, capable of hybridizing with a downstream region of a sequence complementary to the said target sequence, the said downstream region being complementary to the said upstream sequence,
c) and an enzymatic system containing a DNA polymerase activity, an RNA polymerase activity capable of functioning with the said promoter and an activity capable of specifically degrading the region of the target or of the sequence complementary to the target which is capable of pairing with at least part of the second segment of the first or of the second chimeric oligonucleotide, respectively,
and incubating, for a sufficient time, the mixture obtained.

6. Method according to the preceding claim, characterized in that the said degradation activity specific for the target is an RNase H activity.
